(19) **Europäisches Patentamt / European Patent Office / Office européen des brevets**

(11) **EP 2 069 531 B1**

(12) **EUROPEAN PATENT SPECIFICATION**

(45) Date of publication and mention
of the grant of the patent:
**10.04.2013 Bulletin 2013/15**

(21) Application number: **07702476.8**

(22) Date of filing: **05.02.2007**

(51) Int Cl.:
**C12Q 1/68** (2006.01)

(86) International application number:
**PCT/DK2007/000058**

(87) International publication number:
**WO 2007/090399 (16.08.2007 Gazette 2007/33)**

(54) **QTLS FOR MASTITIS RESISTANCE IN CATTLE**

QTLS FÜR MASTITISRESISTENZ BEI RINDERN

RÉSISTANCE À LA MAMMITE

(84) Designated Contracting States:
**AT BE BG CH CY CZ DE DK EE ES FI FR GB GR HU IE IS IT LI LT LU LV MC NL PL PT RO SE SI SK TR**

(30) Priority: **06.02.2006 DK 200600164**
**30.01.2007 DK 200700147**

(43) Date of publication of application:
**17.06.2009 Bulletin 2009/25**

(73) Proprietors:
- **Aarhus Universitet**
  **8000 Aarhus C (DK)**
- **MTT Agrifood Research Finland**
  **31600 Jokioinen (FI)**
- **Estonian University of Life Sciences**
  **51014 Tartu (EE)**

(72) Inventors:
- **LUND, Mogens, Sandø**
  **DK-8830 Tjele (DK)**
- **BENDIXEN, Christian**
  **DK-8860 Ulstrup (DK)**
- **SAHANA, Goutam**
  **DK-8830 Tjele (DK)**
- **SØRENSEN, Peter**
  **DK-8800 Viborg (DK)**
- **SVENDSEN, Søren**
  **DK-8900 Randers (DK)**
- **THOMSEN, Bo**
  **DK-8210 Århus V (DK)**
- **MAJGREN, Bente, Flügel**
  **DK-9500 Hobro (DK)**
- **SABRY, Ayman, Mahmoud**
  **EG-11516 Cairo (EG)**

- **ANDERSSON-EKLUND, Ingrid, Lena**
  **SE-743 40 Storvreta (SE)**
- **VILKKI, Helmi, Johanna**
  **FI-31600 Jokioinen (FI)**
- **ISO-TOURU, Terhi, Katarlina**
  **FI-20700 Turku (FI)**
- **VIITALA, Sirja, Maria**
  **DK-31300 Tammela (FI)**
- **SCHULMAN, Nina, Frederika**
  **DK-32100 Ypäjä (FI)**
- **HASTINGS, Nicola**
  **Midlothian, EH25 9LS (GB)**
- **WILLIAMS, John, Lewis, William**
  **I-26900 Lodi (IT)**
- **WOOLLIAMS, John, Arthur**
  **Peebles EH45 8LF (GB)**
- **ÀVILA, Ana Isabel Fernández**
  **E-28025 Madrid (ES)**
- **VIINALASS, Haldja**
  **Tähtvere vald, Tartu maakond (EE)**
- **VÅRV, Sirje**
  **50408 Tartu (EE)**

(74) Representative: **Høiberg A/S**
**St. Kongensgade 59 A**
**1264 Copenhagen K (DK)**

(56) References cited:
- **HOLMBERG M ET AL: "Quantitative trait loci affecting health traits in Swedish dairy cattle" JOURNAL OF DAIRY SCIENCE, vol. 87, no. 8, August 2004 (2004-08), pages 2653-2659, XP002437633 ISSN: 0022-0302**

- BOICHARD DIDIER ET AL: "Detection of genes influencing economic traits in three French dairy cattle breeds." GENETICS SELECTION EVOLUTION (LES ULIS), vol. 35, no. 1, January 2003 (2003-01), pages 77-101, XP002437634 ISSN: 0999-193X
- MOEMKE S ET AL: "A refined radiation hybrid map of the telomeric region of bovine chromosome 18q25-q26 compared with human chromosome 19q13" ANIMAL GENETICS, vol. 36, no. 2, April 2005 (2005-04), pages 141-145, XP002437194 ISSN: 0268-9146
- KLUNGLAND HELGE ET AL: "Quantitative trait loci affecting clinical mastitis and somatic cell count in dairy cattle" MAMMALIAN GENOME, vol. 12, no. 11, November 2001 (2001-11), pages 837-842, XP002437636 ISSN: 0938-8990
- VILKKI H J ET AL: "Multiple marker mapping of quantitative trait loci of Finnish dairy cattle by regression" JOURNAL OF DAIRY SCIENCE, vol. 80, no. 1, 1997, pages 198-204, XP002437637 ISSN: 0022-0302
- DATABASE EMBL [Online] 13 March 1996 (1996-03-13), "BMS1967 cow Bos taurus STS genomic, sequence tagged site." XP002437656 retrieved from EBI accession no. EMBL:G18713 Database accession no. G18713
- ZHANG Q ET AL: "Mapping quantitative trait loci for milk production and health of dairy cattle in a large outbred pedigree." GENETICS AUG 1998, vol. 149, no. 4, August 1998 (1998-08), pages 1959-1973, XP002450613 ISSN: 0016-6731
- SCHULMAN N F ET AL: "Quantitative trait Loci for health traits in Finnish Ayrshire cattle." JOURNAL OF DAIRY SCIENCE FEB 2004, vol. 87, no. 2, February 2004 (2004-02), pages 443-449, XP002450614 ISSN: 0022-0302
- RODRIGUEZ-ZAS S L ET AL: "Interval and composite interval mapping of somatic cell score, yield, and components of milk in dairy cattle" JOURNAL OF DAIRY SCIENCE, AMERICAN DAIRY SCIENCE ASSOCIATION, SAVOY, IL, US, vol. 85, no. 11, November 2002 (2002-11), pages 3081-3091, XP002437193 ISSN: 0022-0302
- SMARAGDOV M G: "Genetic mapping of loci responsible for milk production traits in dairy cattle" RUSSIAN JOURNAL OF GENETICS, NAUKA/INTERPERIODICA, MO, vol. 42, no. 1, 1 January 2006 (2006-01-01), pages 1-15, XP019301590 ISSN: 1608-3369
- DATABASE BIOSIS [Online] BIOSCIENCES INFORMATION SERVICE, PHILADELPHIA, PA, US August 2008 SAHANA G ET AL: 'Fine-mapping QTL for mastitis resistance on BTA9 in three Nordic red cattle breeds' Database accession no. PREV200800653453 & ANIMAL GENETICS, vol. 39, no. 4, August 2008 (2008-08), pages 354-362, ISSN: 0268-9146, DOI: DOI:10.1111/J. 1365-2052.2008.01729.X

**Description**

**Field of invention**

The present invention relates to a method for determining the resistance to mastitis in a bovine subject comprising detecting at least one genetic marker located on the bovine chromosome BTA9. Furthermore, the present invention relates to use of a diagnostic kit for detecting the presence or absence of at least one genetic marker associated with resistance to mastitis.

**Background of invention**

Mastitis is the inflammation of the mammary gland or udder of the cow resulting from infection or trauma and mastitis is believed to be the most economically important disease in cattle.

The disease may be caused by a variety of agents. The primary cause of mastitis is the invasion of the mammary gland via the teat end by microorganisms.

Mastitis may be clinical or sub-clinical, with sub-clinical infection preceding clinical manifestations. Clinical mastitis (CM) can be detected visually through observing red and swollen mammary glands i.e. red swollen udder, and through the production of clotted milk. Once detected, the milk from mastitic cows is kept separate from the vat so that it will not affect the overall milk quality.

Sub-clinical mastitis is a type of mastitis characterized by high somatic cell counts (SCS), a normal or elevated body temperature, and milk samples that should test positive on culture. Thus, sub-clinical mastitis cannot be detected visually by swelling of the udder or by observation of the gland or the milk produced. Because of this, farmers do not have the option of diverting milk from sub-clinical mastitic cows. However, this milk is of poorer quality than that from non-infected cows and can thus contaminate the rest of the milk in the vat.

Mastitis can be detected by the use of somatic cell counts (SCS) in which a sample of milk from a cow is analysed for the presence of somatic cells (white blood cells). Somatic cells are part of the cow's natural defence mechanism and cell counts rise when the udder becomes infected. The number of somatic cells in a milk sample can be estimated indirectly by rolling-ball viscometer and Coulter counter.

As mastitis results in reduced quantity and quality of milk and products from milk, mastitis results in economic losses to the farmer and dairy industry. Therefore, the ability to determine the genetic basis of resistance to mastitis in a bovine is of immense economic significance to the dairy industry both in terms of daily milk production but also in breeding management, selecting for bovine subjects with resistance to mastitis. A method of genetically selecting bovine subjects with improved resistance that will yield cows less prone to mastitis would be desirable.

One approach to identify genetic determinants for genetic traits is the use of linkage disequilibrium (LD) mapping which aims at exploiting historical recombinants and has been shown in some livestock populations, including dairy cattle, to extend over very long chromosome segments when compared to human populations (Famir et al., 2000). Once mapped, a Quantitative Trait Locus (QTL) can be usefully applied in marker assisted selection.

*Linkage disequilibrium*

Linkage disequilibrium reflects recombination events dating back in history and the use of LD mapping within families increases the resolution of mapping. LD exists when observed haplotypes in a population do not agree with the haplotype frequencies predicted by multiplying together the frequency of individual genetic markers in each haplotype. In this respect the term haplotype means a set of closely linked genetic markers present on one chromosome which tend to be inherited together. In order for LD mapping to be efficient the density of genetic markers needs to be compatible with the distance across which LD extends in the given population. In a study of LD in dairy cattle population using a high number of genetic markers (284 autosomal microsatellite markers) it was demonstrated that LD extends over several tens of centimorgans for intrachromosomal markers (Farnir et al. 2000). Similarly, Georges, M (2000) reported that the location of a genetic marker that is linked to a particular phenotype in livestock typically has a confidence interval of 20-30 cM (corresponding to maybe 500-1000 genes) (Georges, M., 2000). The existence of linkage disequilibrium is taken into account in order to use maps of particular regions of interest with high confidence.

In the present invention quantitative trait loci associated to clinical mastitis and/or SCS have been identified on bovine chromosome BTA9 which allows for a method for determining whether a bovine subject will be resistant to mastitis.

**Summary of invention**

It is of significant economic interest within the cattle industry to be able to select bovine subjects with increased resistance to mastitis and thereby avoid economic losses in connection with animals suffering from mastitis. The genetic predisposition for resistance to mastitis may be detected by the present invention. The present invention offers a method for determining the resistance to mastitis in a bovine subject based on genetic markers which are associated with and/or linked to resistance to mastitis.

Thus, one aspect of the present invention relates to a method for determining the resistance to mastitis in a bovine subject, comprising detecting in a sample from said bovine subject the presence or absence of at least one genetic marker that is linked to at least one trait indicative of mastitis resistance, wherein said at least one genetic marker is located on the bovine chromosome BTA9 in the region flanked by and including the polymorphic microsatellite markers C6orf93 and inra084 ,wherein the presence or absence of said at least one genetic marker is indicative of mastitis resistance of said bovine subject or off-spring therefrom.

A second aspect of the present invention relates to use of a diagnostic kit in detecting the presence or absence in a bovine subject of at least one genetic marker associated with resistance to mastitis, comprising at least one oligonucleotide sequence and combinations thereof, wherein the nucleotide sequences are selected from any of SEQ ID NO.: 75 to SEQ ID NO.: 98.

**Description of Drawings**

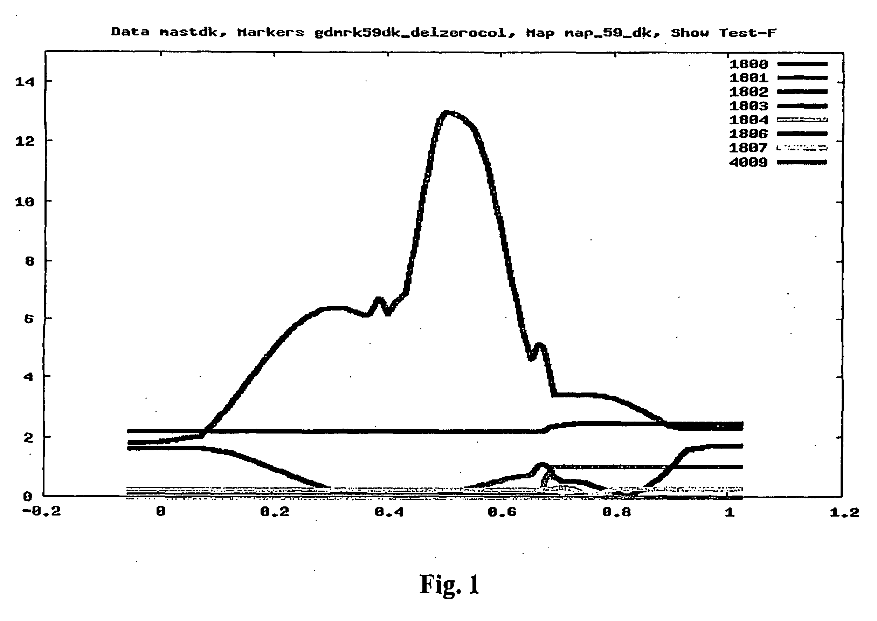

Figure 1: Genome scan of BTA9 in relation to mastitis resistance characteristic of Danish Red families. Numbers refer to 'herdbook number'. The X-axis represents the distance of the chromosome expressed in Morgan according to the positions employed in this analysis. The Y-axis represents the test-statistics of the QTL analysis expressed in the F-value.

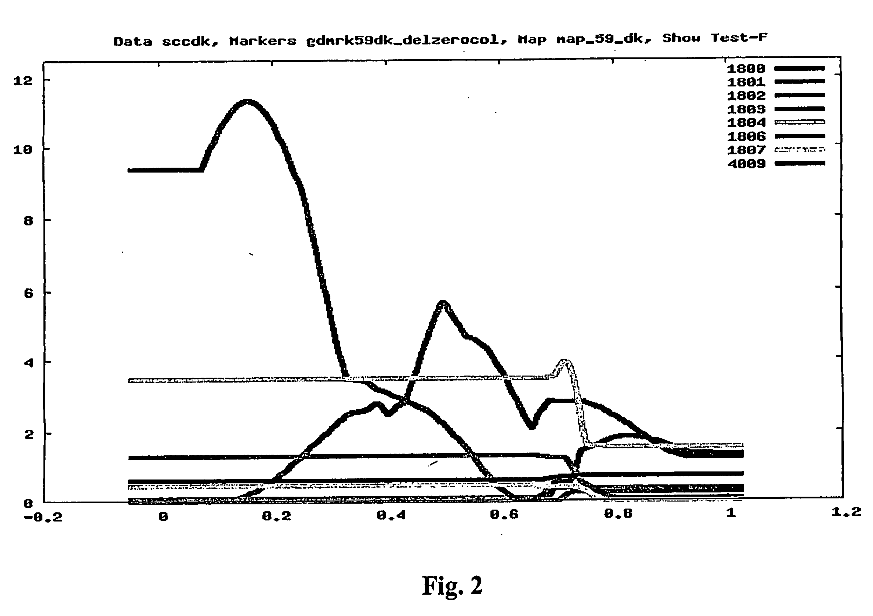

Figure 2: Genome scan of BTA9 in relation to somatic cell count characteristic of Danish Red families. Numbers refer to 'herdbook number'. The X-axis represents the distance of the chromosome expressed in Morgan according to the positions employed in this analysis. The Y-axis represents the test-statistics of the QTL analysis expressed in the F-value.

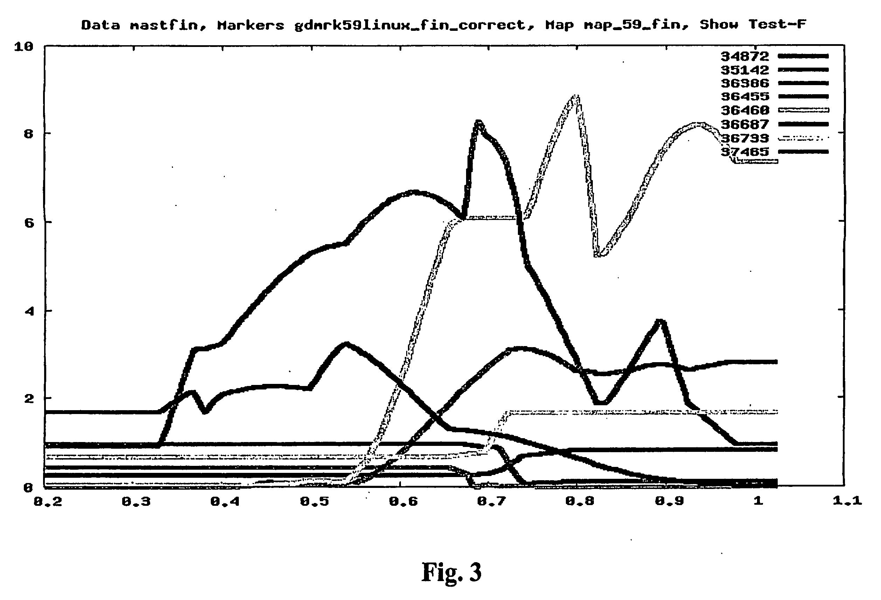

Figure 3: Genome scan of BTA9 in relation to mastitis resistance characteristic of Finnish Ayrshire families. Numbers refer to 'herdbook number'. The X-axis represents the distance of the chromosome expressed in Morgan according to the positions employed in this analysis. The Y-axis represents the test-statistics of the QTL analysis expressed in the F-value.

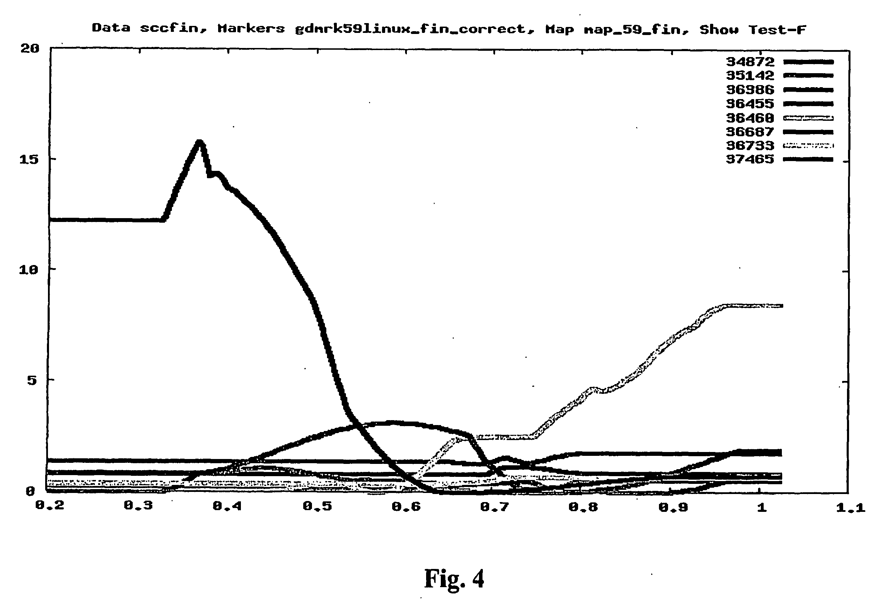

Figure 4: Genome scan of BTA9 in relation to somatic cell count characteristic of Finnish Ayrshire families. Numbers refer to 'herdbook number'. The X-axis represents the distance of the chromosome expressed in Morgan according to the positions employed in this analysis. The Y-axis represents the test-statistics of the QTL analysis expressed in the F-value.

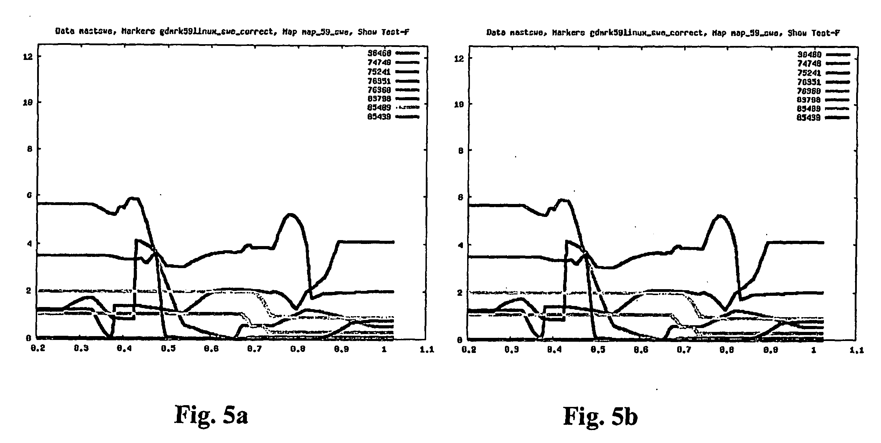

Figure 5a and 5b: Genome scan of BTA9 in relation to mastitis resistance characteristic of Swedish Red and White families. Numbers refer to 'herd book number'. The X-axis represents the distance of the chromosome expressed in Morgan according to the positions employed in this analysis. The Y-axis represents the test-statistics of the QTL analysis expressed in the F-value.

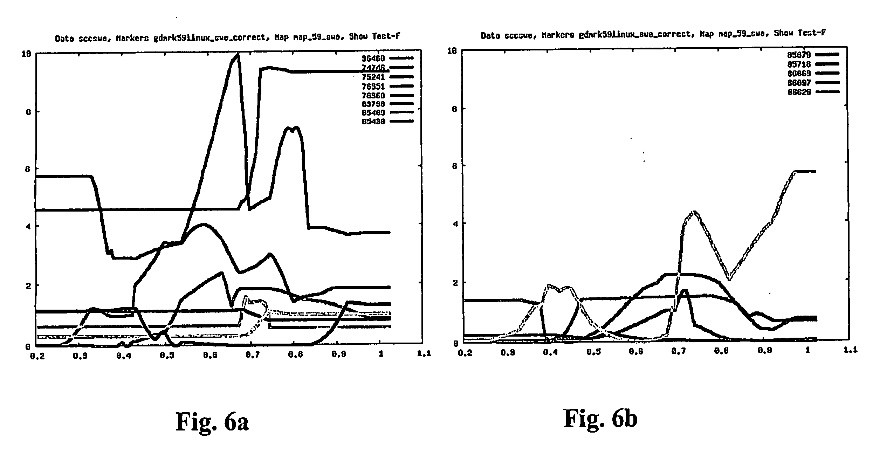

Figure 6a and 6b: Genome scan of BTA9 in relation to somatic cell count characteristic of Swedish Red and White families. Numbers refer to 'herdbook number'. The X-axis represents the distance of the chromosome expressed in Morgan according to the positions employed in this analysis. The Y-axis represents the test-statistics of the QTL analysis expressed in the F-value.

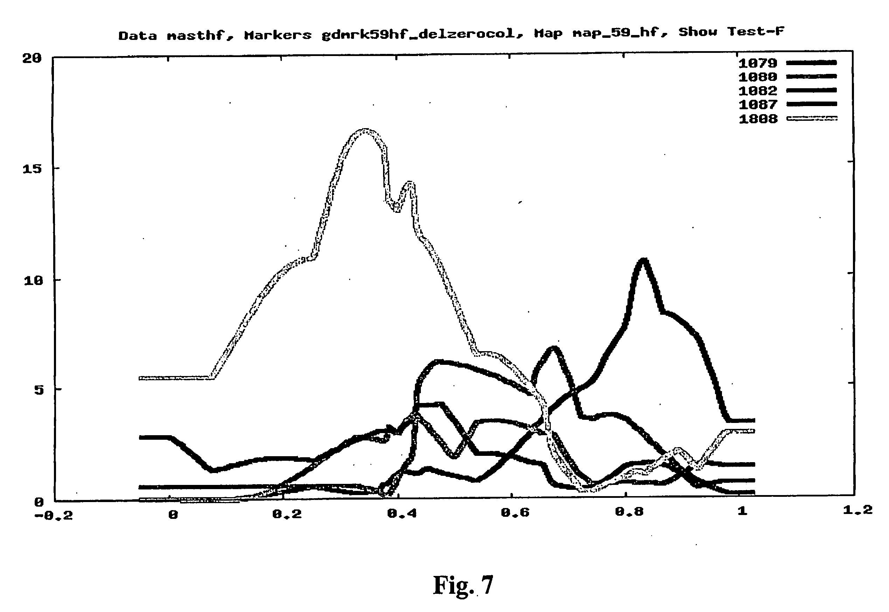

Figure 7: Genome scan of BTA9 in relation to mastitis resistance characteristic of Danish Holstein families. Numbers refer to 'herdbook number'. The X-axis represents the distance of the chromosome expressed in Morgan according to the positions employed in this analysis. The Y-axis represents the test-statistics of the QTL analysis expressed in the F-value.

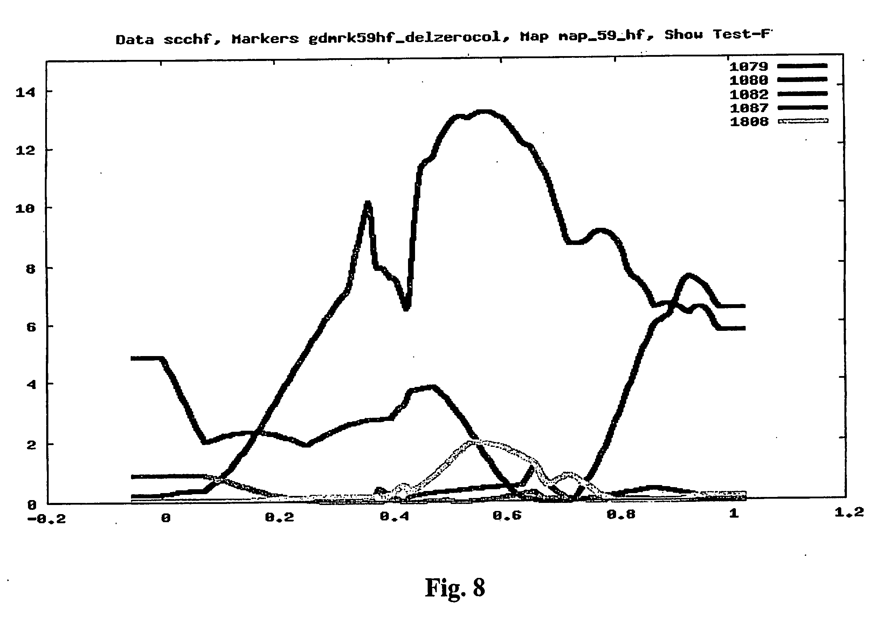

Figure 8: Genome scan of BTA9 in relation to somatic cell count characteristic of Danish Holstein families. Numbers refer to 'herdbook number'. The X-axis represents the distance of the chromosome expressed in Morgan according to the positions employed in this analysis. The Y-axis represents the test-statistics of the QTL analysis expressed in the F-value.

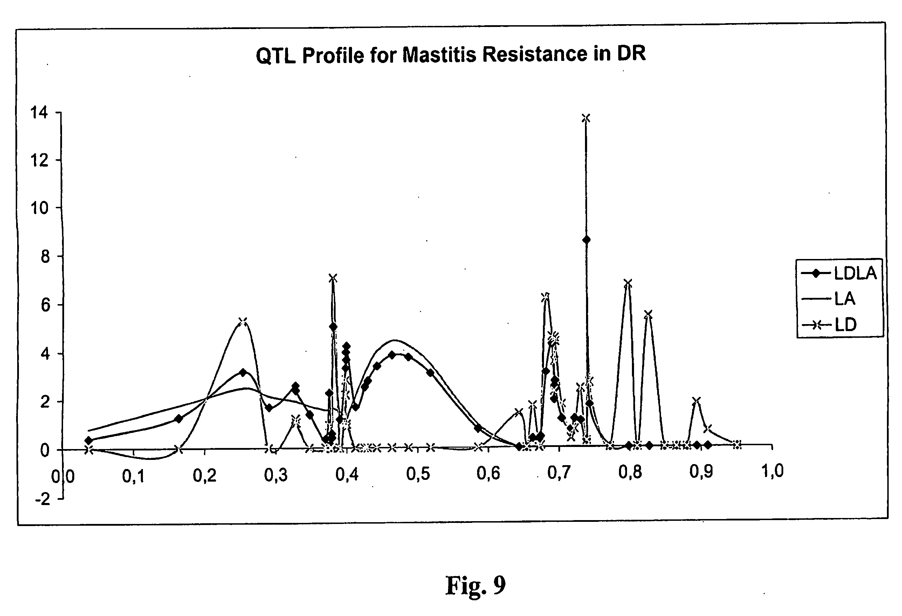

Figure 9: QTL profile for the trait mastitis resistance showing LDLA/LD peak between the markers BMS2819 and INRA144 at 74.08 cM in Danish Red cattle. The X-axis represents the distance of the chromosome expressed in Morgan according to the positions employed in this analysis. The Y-axis represents the likelihood ratio test-statistics of the QTL analysis.

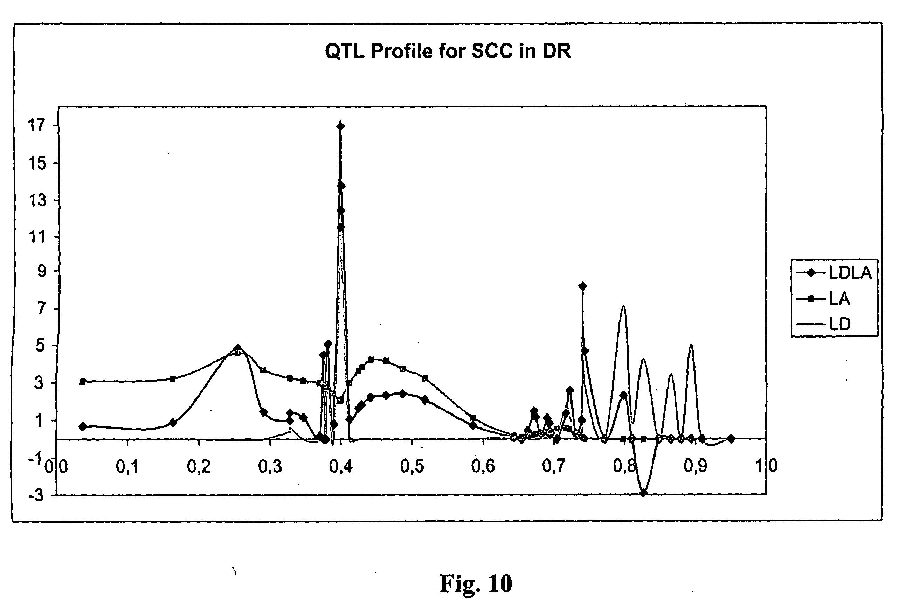

Figure 10: QTL profile for the trait Somatic Cell Count showing LDLA peak between the markers BMS2819 and INRA144 at 74.075 cM in Danish Red cattle. The X-axis represents the distance of the chromosome expressed in Morgan according to the positions employed in this analysis. The Y-axis represents the likelihood ratio test-statistics of the QTL analysis.

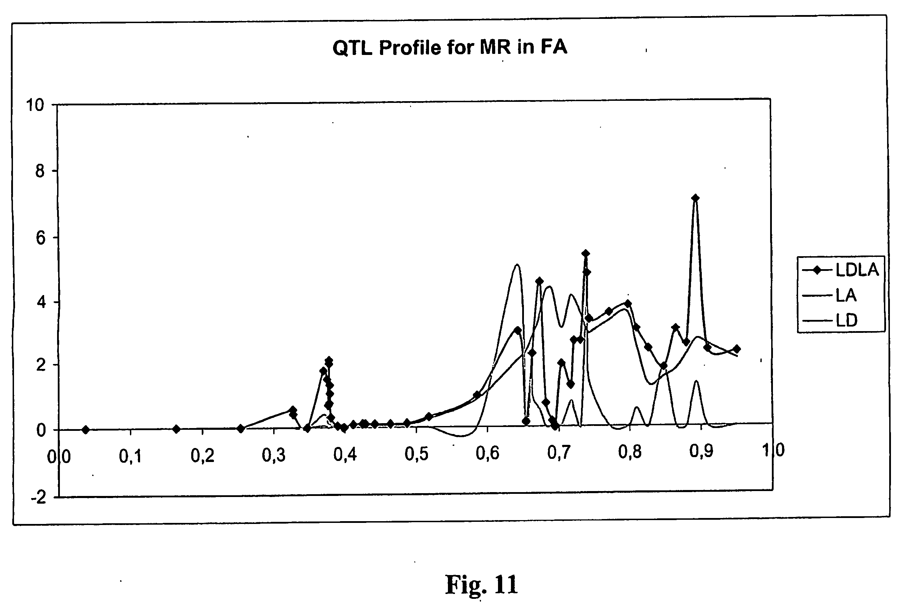

Figure 11: QTL profile for the trait mastitis resistance showing LDLA peak between the markers BM4208 and INRA144 in Finnish Ayrshire breed. The X-axis represents the distance of the chromosome expressed in Morgan according to the positions employed in this analysis. The Y-axis represents the likelihood ratio test-statistics of the QTL analysis.

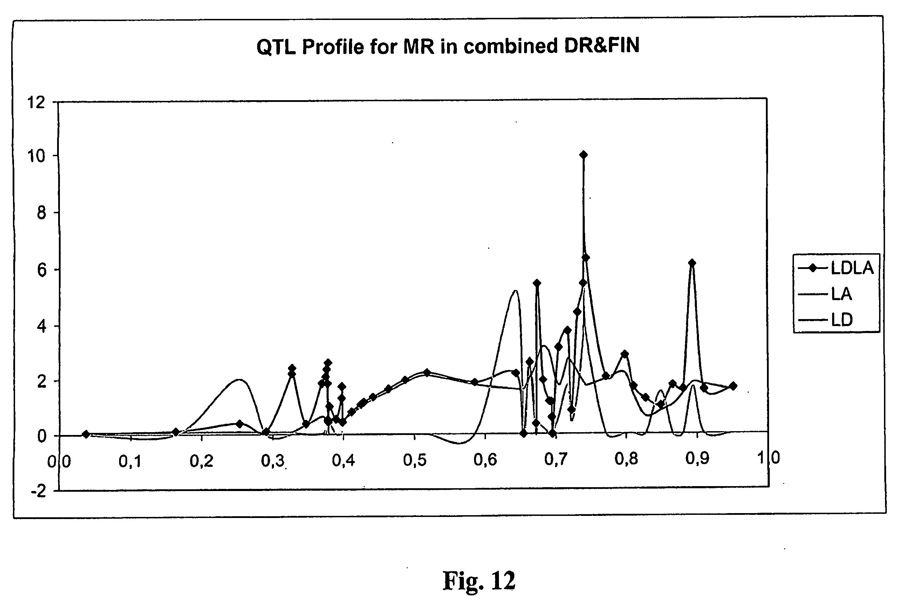

Figure 12: QTL profile for the trait mastitis resistance showing LDLA/LD peak between the markers BMS2819 and INRA144 in combined Finnish Ayrshire and Danish Red cattle. The X-axis represents the distance of the chromosome expressed in Morgan according to the positions employed in this analysis. The Y-axis represents the likelihood ratio test-statistics of the QTL analysis.

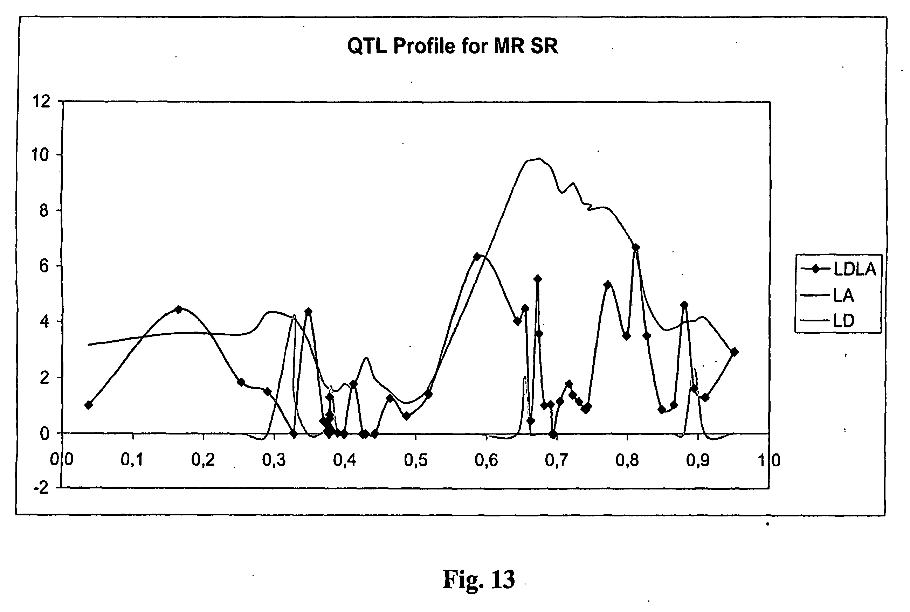

Figure 13: QTL profile for the trait mastitis resistance showing LA peak at 60 - 80 cM markers BMS2819 and INRA144 in Swedish Red and White cattle. The X-axis represents the distance of the chromosome expressed in Morgan according to the positions employed in this analysis. The Y-axis represents the likelihood ratio test-statistics of the QTL analysis.

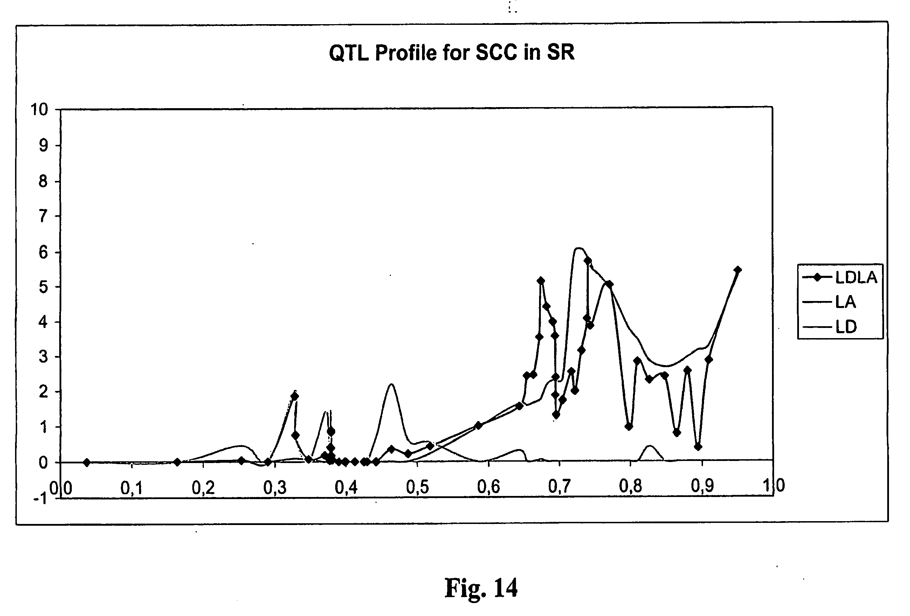

Figure 14: QTL profile for SCS in Swedish Red and White cattle with LDLA evidence between markers BMS2819 and INRA084. The X-axis represents the distance of the chromosome expressed in Morgan according to the positions employed in this analysis. The Y-axis represents the likelihood ratio test-statistics of the QTL analysis.

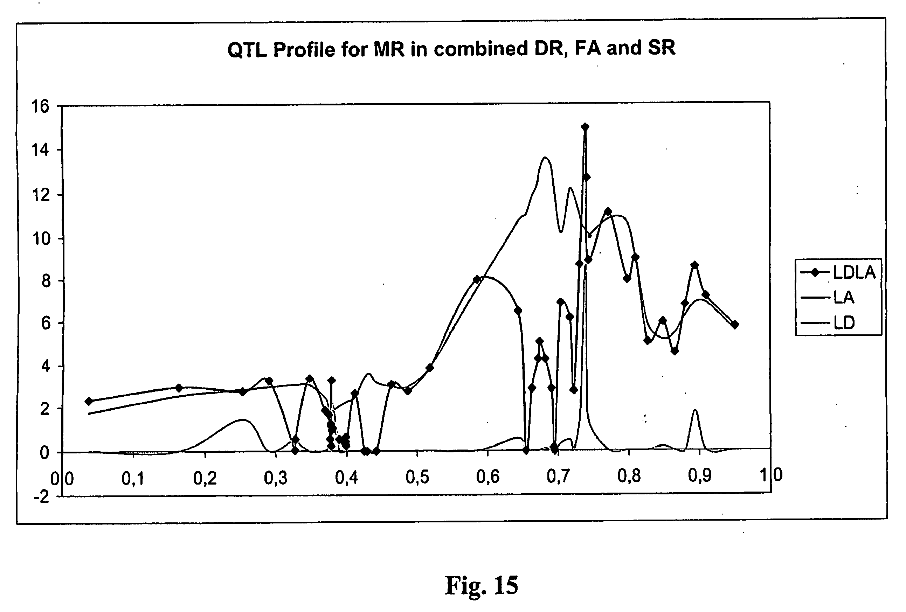

Figure 15: QTL profile for the trait mastitis resistance showing LDLA/LD peak between the markers BM4208 and INRA144 in combined analyses of Finnish Ayrshire, Danish Red cattle and Swedish Red and White cattle. The X-axis represents the distance of the chromosome expressed in Morgan according to the positions employed, in this analysis. The Y-axis represents the likelihood ratio test-statistics of the QTL analysis.

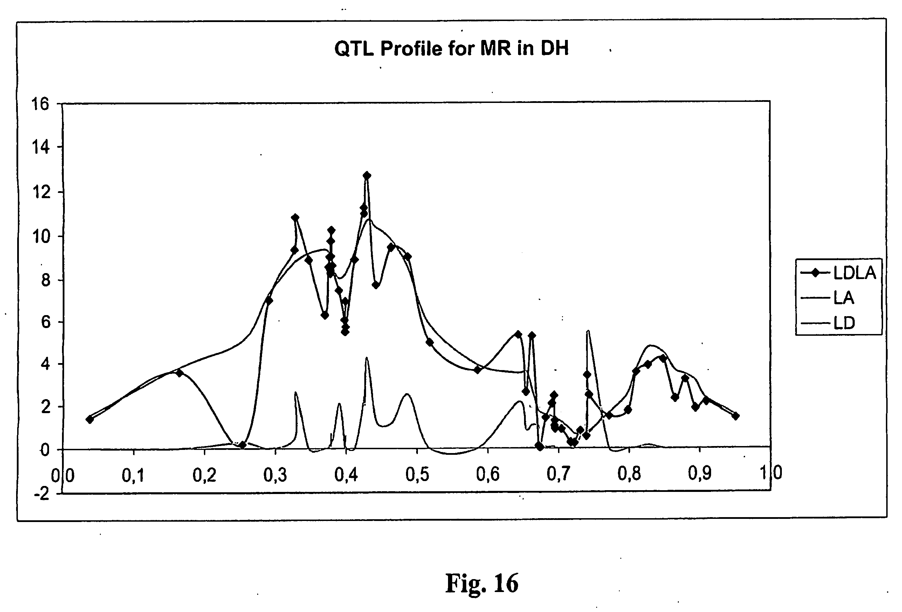

Figure 16: QTL profile for the trait mastitis resistance Danish Holstein cattle. The X-axis represents the distance of the chromosome expressed in Morgan according to the positions employed in this analysis. The Y-axis represents the likelihood ratio test-statistics of the QTL analysis.

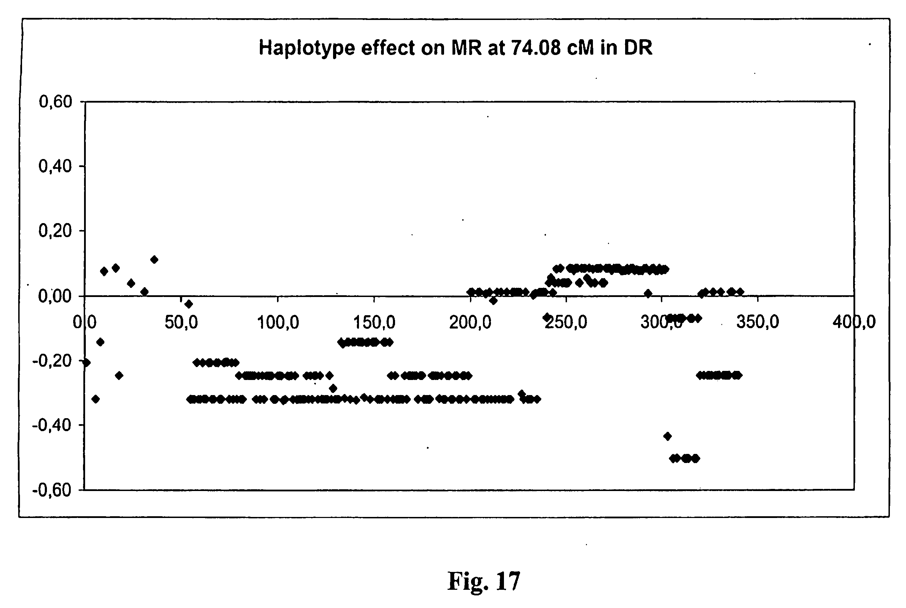

Figure 17: The haplotypes effect on mastitis resistance at 74.08 cM in Danish Red. The haplotypes effects are on the y-axis and haplotypes number is on x-axis. In the beginning there are the large clusters of dam haplotypes followed by the sire haplotypes.

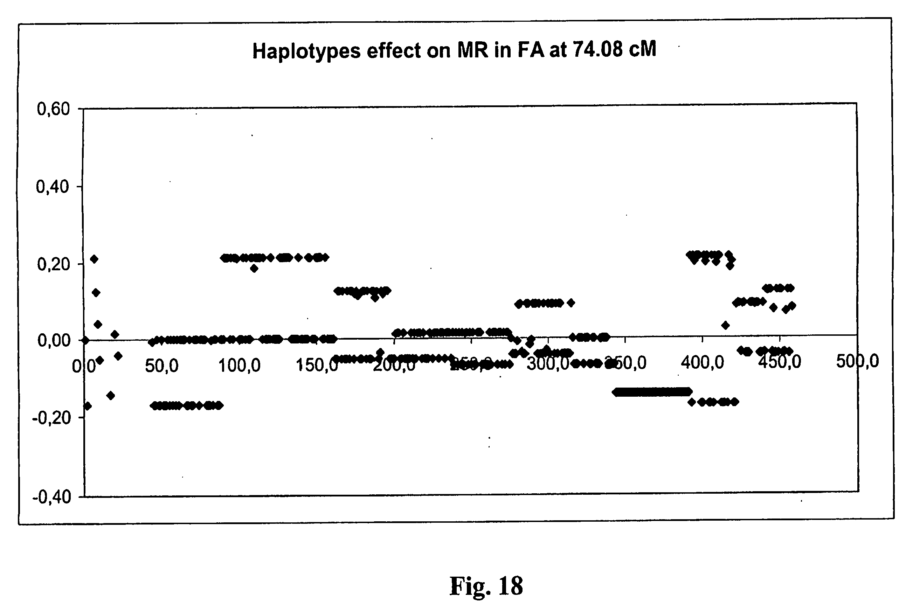

Figure 18: The haplotypes effect on mastitis resistance at 74.08 cM in Finnish Ayrshire. The haplotypes effects are on the y-axis and haplotypes number is on x-axis. In the beginning there are the large clusters of dam haplotypes followed by the sire haplotypes.

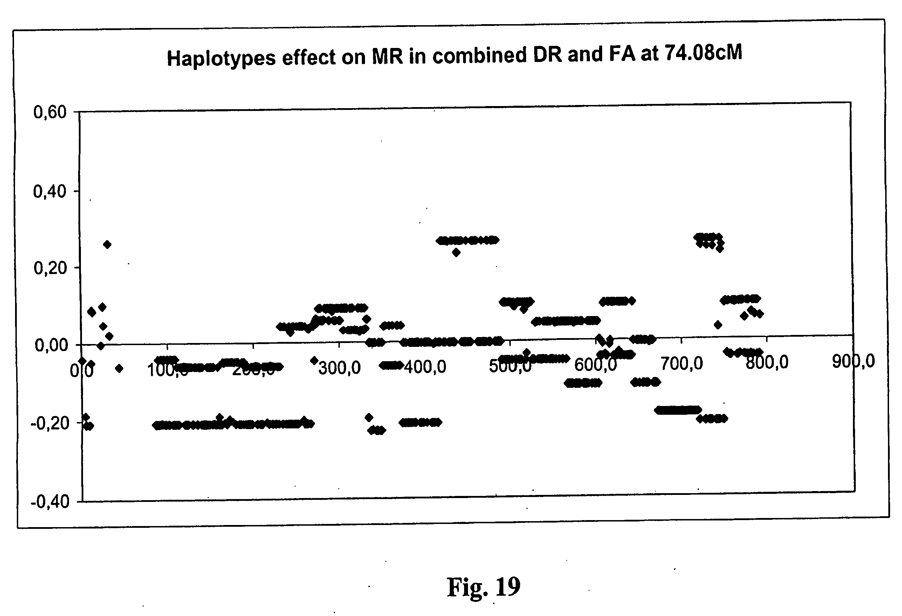

Figure 19: The haplotypes effect on mastitis resistance at 74.08 cM in combined Danish Red and Finnish Ayrshire. The haplotypes effects are on the y-axis and haplotypes number is on x-axis. In the beginning there are the large clusters of dam haplotypes followed by the sire haplotypes.

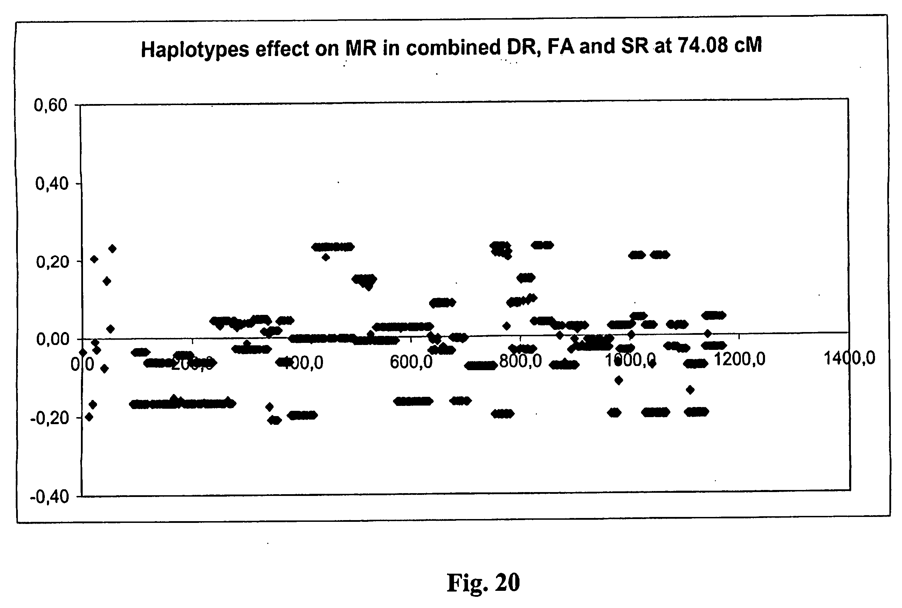

Figure 20: The haplotypes effect on mastitis resistance at 74.08 cM in combined Danish Red, Finnish Ayrshire and Swedish Red and White. The haplotypes effects are on the y-axis and haplotypes number is on x-axis. In the beginning there are the large clusters of dam haplotypes followed by the sire haplotypes.

## Detailed description of the invention

The present invention relates to genetic determinants of mastitis resistance in dairy cattle. The occurrence of mastitis, both clinical and sub-clinical mastitis involves substantial economic loss for the dairy industry. Therefore, it is of economic interest to identity those bovine subjects that have a genetic predisposition for mastitis resistance. Bovine subjects with such genetic predisposition are carriers of desired traits, which can be passed on to their offspring.

The term "bovine subject" refers to cattle of any breed and is meant to include both cows and bulls, whether adult or newborn animals. No particular age of the animals are denoted by this term. One example of a bovine subject is a member of the Holstein breed. In one preferred embodiment, the bovine subject is a member of the Holstein-Friesian cattle population. In another embodiment, the bovine subject is a member of the Holstein Swartbont cattle population. In another embodiment, the bovine subject is a member of the Deutsche Holstein Schwarzbunt cattle population. In another embodiment, the bovine subject is a member of the US Holstein cattle population. In one embodiment, the bovine subject is a member of the Red and White Holstein breed. In another embodiment, the bovine subject is a member of the Deutsche Holstein Schwarzbunt cattle population. In one embodiment, the bovine subject is a member of any family, which include members of the Holstein breed. In one preferred embodiment the bovine subject is a member of the Danish Red population. In another preferred embodiment the bovine subject is a member of the Finnish Ayrshire population. In yet another embodiment the bovine subject is a member of the Swedish Red and White population. In a further embodiment the bovine subject is a member of the Danish Holstein population. In another embodiment, the bovine subject is a member of the Swedish Red and White population. In yet another embodiment, the bovine subject is a member of the Nordic Red population.

In one embodiment of the present invention, the bovine subject is selected from the group consisting of Swedish Red and White, Danish Red, Finnish Ayrshire, Holstein-Friesian, Danish Holstein and Nordic Red. In another embodiment of the present invention, the bovine subject is selected from the group consisting of Finnish Ayrshire and Swedish Red and White cattle. In another embodiment of the present invention, the bovine subject is selected from the group consisting of Finnish Ayrshire and Swedish Red and White cattle.

The term "genetic marker" refers to a variable nucleotide sequence (polymorphism) of the DNA on the bovine chromosome and distinguishes one allele from another. The variable nucleotide sequence can be identified by methods known to a person skilled in the art for example by using specific oligonucleotides in for example amplification methods and/or observation of a size difference. However, the variable nucleotide sequence may also be detected by sequencing or for example restriction fragment length polymorphism analysis. The variable nucleotide sequence may be represented by a deletion, an insertion, repeats, and/or a point mutation.

One type of genetic marker is a microsatellite marker that is linked to a quantitative trait locus. Microsatellite markers refer to short sequences repeated after each other. In short sequences are for example one nucleotide, such as two nucleotides, for example three nucleotides, such as four nucleotides, such as five nucleotides, such as six nucleotide, for example seven nucleotides, such as eight nucleotides, for example nine nucleotides, such as ten nucleotides. However, changes sometimes occur and the number of repeats may increase or decrease. The specific definition and locus of the polymorphic microsatellite markers can be found in the USDA genetic map (Kappes et al. 1997; or by following the link to U.S. Meat Animal Research Center http://www.marc.usda.gov/).

It is furthermore appreciated that the nucleotide sequences of the genetic markers of the present invention are genetically linked to traits for mastitis resistance in a bovine subject. Consequently, it is also understood that a number of genetic markers may be generated from the nucleotide sequence of the DNA region(s) flanked by and including the genetic markers according to the method of the present invention.

The term 'Quantitative trait locus (QTL)' is a region of DNA that is associated with a particular trait (e.g., plant height). Though not necessarily genes themselves, QTLs are stretches of DNA that are closely linked to the genes that underlie the trait in question.

The term 'mastitis' is in the present application used to describe both the sub-clinical mastitis characterized for example by high somatic cell score (SCS) and clinical mastitis.

The terms 'mastitis resistance' and 'resistance to mastitis' are used interchangeable and relates to the fact that some bovine subjects are not as prone to mastitis as are other bovine subjects. When performing analyses of a number of bovine subjects as in the present invention in order to determine genetic markers that are associated with resistance to mastitis, the traits implying resistance to mastitis may be observed by the presence or absence of genetic markers linked to occurrence of clinical mastitis and/or sub-clinical mastitis in the bovine subjects analyzed. It is understood that mastitis resistance comprise resistance to traits, which affect udder health in the bovine subject or its off-spring. Thus, mastitis resistance of a bull is physically manifested by its female off-spring.

**Scoring for mastitis resistance**

Daughters of bulls were scored for mastitis resistance and SCC. Somatic cell score (SCS) was defined as the mean of $\log^{10}$ transformed somatic cell count values (in 10,000/mL) obtained from the milk recording scheme. The mean was taken over the period 10 to 180 after calving. Estimated breeding values (EBV) for traits of sons were calculated using a single trait Best Linear Unbiased Prediction (BLUP) animal model ignoring family structure. These EBVs were used in the QTL analysis. The daughter registrations used in the individual traits were:

Clinical mastitis in Denmark: Treated cases of clinical mastitis in the period -5 to 50 days after 1st calving.
Clinical mastitis in Sweden and Finland: Treated cases of clinical mastitis in the period -7 to 150 days after 1st calving.
SCS in Denmark: Mean SCS in period 10-180 days after 1st calving.
SCS in Sweden: Mean SCS in period 10-150 days after 1st calving.
SCS in Finland: Mean SCS in period 10-305 days after 1st calving.

In one embodiment of the present invention, the method and kit described herein relates to mastitis resistance. In another embodiment of the present invention, the method and kit described herein relates to resistance to clinical mastitis. In another embodiment, the method and kit of the present invention pertains to resistance to sub-clinical mastitis, such as detected by somatic cell counts. In yet another embodiment, the method and kit of the present invention primarily relates to resistance to clinical mastitis in combination with resistance to sub-clinical mastitis such as detected by somatic cell counts.

## Sample

The method according to the present invention includes analyzing a sample of a bovine subject, wherein said sample may be any suitable sample capable of providing the bovine genetic material for use in the method. The bovine genetic material may for example be extracted, isolated and purified if necessary from a blood sample, a tissue samples (for example spleen, buccal smears), clipping of a body surface (hairs or nails), milk and/or semen. The samples may be fresh or frozen.

The sequence polymorphisms of the invention comprise at least one nucleotide difference, such as at least two nucleotide differences, for example at least three nucleotide differences, such as at least four nucleotide differences, for example at least five nucleotide differences, such as at least six nucleotide differences, for example at least seven nucleotide differences, such as at least eight nucleotide differences, for example at least nine nucleotide differences, such as 10 nucleotide differences. The nucleotide differences comprise nucleotide differences, deletion and/or insertion or any combination thereof.

## Grand daughter design

The grand daughter design includes analysing data from DNA-based markers for grand sires that have been used extensively in breeding and for sons of grand sires where the sons have produced offspring. The phenotypic data that are to be used together with the DNA-marker data are derived from the daughters of the sons. Such phenotypic data could be for example milk production features, features relating to calving, meat quality, or disease. One group of daughters have inherited one allele from their father whereas a second group of daughters have inherited the other allele form their father. By comparing data from the two groups information can be gained whether a fragment of a particular chromosome is harbouring one or more genes that affect the trait in question. It may be concluded whether a QTL is present within this fragment of the chromosome.

A prerequisite for performing a grand daughter design is the availability of detailed phenotypic data. In the present invention such data have been available ( http://www.lr.dk/kvaeg/diverse/principles.pdf ).

QTL is a short form of quantitative trait locus. Genes conferring quantitative traits to an individual may be found in an indirect manner by observing pieces of chromosomes that act as if one or more gene(s) is located within that piece of the chromosome.

In contrast, DNA markers can be used directly to provide information of the traits passed on from parents to one or more of their off spring when a number of DNA markers on a chromosome have been determined for one or both parents and their off-spring. The markers may be used to calculate the genetic history of the chromosome linked to the DNA markers.

## Chromosomal regions and markers

BTA is short for *Bos taurus* autosome.

One aspect of the present invention relates to a method for determining the resistance to mastitis in a bovine subject, comprising detecting in a sample from said bovine subject the presence or absence of at least one genetic marker that is linked to at least one trait indicative of mastitis resistance, wherein said at least one genetic marker is located on the bovine chromosome BTA9 in the region flanked by and including the polymorphic microsatellite markers C6orf93 and inra084 , wherein the presence or absence of said at least one genetic marker is indicative of mastitis resistance of said bovine subject or off-spring therefrom.

Due to the concept of linkage disequilibrium as described herein the present invention also relates to determining the resistance to mastitis in a bovine subject, wherein the at least one genetic marker is linked to a bovine trait for resistance to mastitis.

In order to determine resistance to mastitis in a bovine subject, it is appreciated that more than one genetic marker may be employed in the present invention. For example the at least one genetic marker may be a combination of at least two or more genetic markers such that the accuracy may be increased, such as at least three genetic markers, for example four genetic markers, such as at least five genetic markers, for example six genetic markers, such as at least seven genetic markers, for example eight genetic markers, such as at least nine genetic markers, for example ten genetic markers.

The at least one genetic marker may be located on at least one bovine chromosome, such as two chromosomes, for example three chromosomes, such as four chromosomes, for example five chromosomes, and/or such as six chromosomes. The at least one genetic marker may be located on the bovine chromosome 9. However, the at least one genetic marker may a combination of markers located on different chromosomes.

The at least one genetic marker is selected from any of the individual markers of the tables shown herein.

According to the invention the at least one genetic marker is located on the bovine chromosome BTA9 in the region flanked by and including the markers c6orf93 and inra084. In one embodiment of the present invention, the at least one genetic marker is located in the region from about. 69.35 cM to about 79.8 cM (according to the positions employed in this analysis) on the bovine chromosome BTA9. The at least one genetic marker is selected from the group of markers shown in Table1

Table 1

| Marker on BTA9 | Position employed in analysis (cM) | Relative position (cM) http://www.marc.usda.gov/ |
|---|---|---|
| C6orf93* | 69.35 | - |
| DIK 4986 | 69.4 | 84.258 |
| mm12e6* | 69.45 | 84.258 |
| PEX3* | 69.5 | - |
| DEAD21 | 69.55 | - |
| BMS2251 | 71.3 | 86.58 |
| EPM2A* | 72.1 | |
| BM7234 | 72.3 | 88.136 |
| BM42U8 | 73.9 | 90.69 |
| BMS2819 | 73.95 | 90.98 |
| INRA144 | 74.2 | 90.98 |
| INRA084 | 74.5 | 90.98 |
| * denotes markers that are not listed on the MARC marker map at BTA9. | | |

In another embodiment of the invention the at least one genetic marker is located on the bovine chromosome BTA9 in the region flanked by and including the markers c6orf93 and inra084. In one embodiment of the present invention, the at least one genetic marker is located in the region from about 69.35 cM to about 74.5 cM (according to the positions employed in this analysis) on the bovine chromosome BTA9. According to the MARC marker map the position of the genetic marker inra084 is 90.98. The at least one genetic marker is selected from the group of markers shown in Table 2.

Table 2

| Marker on BTA9 | Position employed in analysis (cM) | Relative position (cM) http://www.marc.usda.gov/ |
|---|---|---|
| C6orf93* | 69.35 | - |
| DIK 4986 | 69.4 | 84.258 |
| mm12e6* | 69.45 | 84.258 |
| PEX3* | 69.5 | - |
| DEAD21 | 69.55 | - |
| BMS2251 | 71.3 | 86.58 |

(continued)

| Marker on BTA9 | Position employed in analysis (cM) | Relative position (cM) http://www.marc.usda.gov/ |
|---|---|---|
| EPM2A* | 72.1 | |
| BM7234 | 72.3 | 88.136 |
| BM4208 | 73.9 | 90.69 |
| BMS2819 | 73.95 | 90.98 |
| INRA144 | 74.2 | 90.98 |
| INRA084 | 74.5 | 90.98 |
| * denotes markers that are not listed on the MARC marker map at BTA9. | | |

In a further embodiment the at least one genetic marker is located on the bovine chromosome BTA9 in the region flanked by and including the markers bms2251 and inra 084. In one embodiment of the present invention, the at least one genetic marker is located in the region from about 71.3 cM to about 74.5 cM on the bovine chromosome BTA9 (according to the positions employed in this analysis) and from about 86.58 cM to about 90.98 cM according to the to the MARC marker map. The at least one genetic marker is selected from the group of markers shown in Table 3.

Table 3

| Marker on BTA9 | Position employed in analysis (cM) | Relative position (cM) http://www.marc.usda.gov/ |
|---|---|---|
| BMS2251 | 71.3 | 86.58 |
| EPM2A* | 72.1 | |
| BM7234 | 72.3 | 88.136 |
| BM4208 | 73.9 | 90.69 |
| BMS2819 | 73.95 | 90.98 |
| INRA144 | 74.2 | 90.98 |
| INRA084 | 74.5 | 90.98 |
| * denotes markers that are not listed on the MARC marker map at BTA9. | | |

In another embodiment the at least one genetic marker is located on the bovine chromosome BTA9 in the region flanked by and including the markers bms2251 and inra144. In one embodiment of the present invention, the at least one genetic marker is located in the region from about 71.3 cM to about 74.2 cM on the bovine chromosome BTA9 (according to the positions employed in this analysis) and from about 86.58 cM to about 90.98 cM according to the to the MARC marker map. The at least one genetic marker is selected from the group of markers shown in Table 4.

Table 4

| Marker on BTA9 | Position employed in analysis (cM) | Relative position (cM) http://www.marc.usda.gov/ |
|---|---|---|
| BMS2251 | 71.3 | 86.58 |
| EPM2A* | 72.1 | |
| BM7234 | 72.3 | 88.136 |
| BM4208 | 73.9 | 90.69 |
| BMS2819 | 73.95 | 90.98 |
| INRA144 | 74.2 | 90.98 |
| * denotes markers that are not listed on the MARC marker map at BTA9. | | |

In yet another embodiment the at least one genetic marker is located on the bovine chromosome BTA9 in the region flanked by and including the markers bm7234 and inra084. In one embodiment of the present invention, the at least one genetic marker is located in the region from about 72.3 cM to about 74.5 cM on the bovine chromosome BTA9 (according to the positions employed in this analysis) and from about 88.136 cM to about 90.98 cM according to the to the MARC marker map. The at least one genetic marker is selected from the group of markers shown in Table 5.

Table 5

| Marker on BTA9 | Position employed in analysis (cM) | Relative position (cM) http://www.marc.usda.gov/ |
|---|---|---|
| BM7234 | 72.3 | 88.136 |
| BM4208 | 73.9 | 90.69 |
| BMS2819 | 73.95 | 90.98 |
| INRA144 | 74.2 | 90.98 |
| INRA084 | 74.5 | 90.98 |
| * denotes markers that are not listed on the MARC marker map at BTA9. | | |

In a further embodiment the at least one genetic marker is located on the bovine chromosome BTA9 in the region flanked by and including the markers bm7234 and inra144. In one embodiment of the present invention, the at least one genetic marker is located in the region from about 72.3 cM to about 74.2 cM on the bovine chromosome BTA9 (according to the positions employed in this analysis) and from about 88.136 cM to about 90.98 cM according to the to the MARC marker map. The at least one genetic marker is selected from the group of markers shown in Table 6.

Table 6

| Marker on BTA9 | Position employed in analysis (cM) | Relative position (cM) http://www.marc.usda.gov/ |
|---|---|---|
| BM7234 | 72.3 | 88.136 |
| BM4208 | 73.9 | 90.69 |
| BMS2819 | 73.95 | 90.98 |
| INRA144 | 74.2 | 90.98 |
| * denotes markers that are not listed on the MARC marker map at BTA9. | | |

In yet a further embodiment the at least one genetic marker is located on the bovine chromosome BTA9 in the region flanked by and including the markers bm7234 and bms2819. In one embodiment of the present invention, the at least one genetic marker is located in the region from about 72.3 cM to about 73.95 cM on the bovine chromosome BTA9 (according to the positions employed in this analysis) and from about 88.136 cM to about 90.98 cM according to the to the MARC marker map. The at least one genetic marker is selected from the group of markers shown in Table 7.

Table 7

| Marker on BTA9 | Position employed in analysis (cM) | Relative position (cM) http://www.marc.usda.gov/ |
|---|---|---|
| BM7234 | 72.3 | 88.136 |
| BM4208 | 73.9 | 90.69 |
| BMS2819 | 73.95 | 90.98 |
| * denotes markers that are not listed on the MARC marker map at BTA9. | | |

In another embodiment the at least one genetic marker is located on the bovine chromosome BTA9 in the region flanked by and including the markers bm7234 and bm4208. In one embodiment of the present invention, the at least one genetic marker is located in the region from about 72.3 cM to about 73.9 cM on the bovine chromosome BTA9 (according to the positions employed in this analysis) and from about 88.136 cM to about 90.69 cM according to the to the MARC marker map. The at least one genetic marker is selected from the group of markers shown in Table 8.

Table 8

| Marker on BTA9 | Position employed in analysis (cM) | Relative position (cM) http://www.marc.usda.gov/ |
|---|---|---|
| BM7234 | 72.3 | 88.136 |
| BM4208 | 73.9 | 90.69 |
| * denotes markers that are not listed on the MARC marker map at BTA9. | | |

In a further embodiment the at least one genetic marker is located on the bovine chromosome BTA9 in the region flanked by and including the markers bms2819 and inra144. In one embodiment of the present invention, the at least one genetic marker is located in the region from about 73.95 cM to about 74.2 cM on the bovine chromosome BTA9 (according to the positions employed in this analysis) and from about 90.98 cM to about 90.98 cM according to the to the MARC marker map. The at least one genetic marker is selected from the group of markers shown in Table 9.

Table 9

| Marker on BTA9 | Position employed in analysis (cM) | Relative position (cM) http://www.marc.usda.gov/ |
|---|---|---|
| BMS2819 | 73.95 | 90.98 |
| INRA144 | 74.2 | 90.98 |
| * denotes markers that are not listed on the MARC marker map at BTA9. | | |

In another embodiment of the present invention the at least one genetic marker is located on the bovine chromosome BTA9 in the region flanked by and including the markers bms2819 and inra084. In one embodiment of the present invention, the at least one genetic marker is located in the region from about 73.95 cM to about 74.5 cM on the bovine chromosome BTA9 (according to the positions employed in this analysis) and from about 90.98 cM to about 90.98 cM according to the to the MARC marker map. The at least one genetic marker is selected from the group of markers shown in Table 10.

Table 10

| Marker on BTA9 | Position employed in analysis (cM) | Relative position (cM) http://www.marc.usda.gov/ |
|---|---|---|
| BMS2819 | 73.95 | 90.98 |
| INRA144 | 74.2 | 90.98 |
| INRA084 | 74.5 | 90.98 |
| * denotes markers that are not listed on the MARC marker map at BTA9. | | |

In another embodiment of the present invention the at least one genetic marker is located on the bovine chromosome BTA9 in the region flanked by and including the markers bm4208 and inra144. In one embodiment of the present invention, the at least one genetic marker is located in the region from about 73.9 cM to about 74.2 cM on the bovine chromosome BTA9 (according to the positions employed in this analysis) and from about 90.69 cM to about 90.98 cM according to the to the MARC marker map. The at least one genetic marker is selected from the group of markers shown in Table 11.

Table 11

| Marker on BTA9 | Position employed in analysis (cM) | Relative position (cM) http://www.marc.usda.gov/ |
|---|---|---|
| BM4208 | 73.9 | 90.69 |
| BMS2819 | 73.95 | 90.98 |
| INRA144 | 74.2 | 90.98 |
| INRA084 | 74.5 | 90.98 |
| * denotes markers that are not listed on the MARC marker map at BTA9. | | |

In yet another embodiment of the present invention the at least one genetic marker is located on the bovine chromosome BTA9 in the region flanked by and including the markers inra144 and inra084. In one embodiment of the present invention, the at least one genetic marker is located in the region from about 74.2 cM to about 74.5 cM on the bovine chromosome BTA9 (according to the positions employed in this analysis) and from about 90.98 cM to about 90.98 cM according to the to the MARC marker map. The at least one genetic marker is selected from the group of markers shown in Table 12.

Table 12

| Marker on BTA9 | Position employed in analysis (cM) | Relative position (cM) http://www.marc.usda.gov/ |
| --- | --- | --- |
| INRA144 | 74.2 | 90.98 |
| INRA084 | 74.5 | 90.98 |
| * denotes markers that are not listed on the MARC marker map at BTA9. | | |

In a further embodiment the at least one genetic marker is located on the bovine chromosome BTA9 in the region flanked by and including the markers bms2251 and bm7234. In one embodiment of the present invention, the at least one genetic marker is located in the region from about 71.3 cM to about 72.3 cM on the bovine chromosome BTA9 (according to the positions employed in this analysis) and from about 86.58 cM to about 88.136 cM according to the to the MARC marker map. The at least one genetic marker is selected from the group of markers shown in Table 13.

Table 13

| Marker on BTA9 | Position employed in analysis (cM) | Relative position (cM) http://www.marc.usda.gov/ |
| --- | --- | --- |
| BMS2251 | 71.3 | 86.58 |
| EPM2A* | 72.1 | |
| BM7234 | 72.3 | 88.136 |
| * denotes markers that are not listed on the MARC marker map at BTA9. | | |

In yet a further embodiment the at least one genetic marker is located on the bovine chromosome BTA9 in the region flanked by and including the markers EPM2A and bm7234. In one embodiment of the present invention, the at least one genetic marker is located in the region from about 72.1 cM to about 72.3 cM on the bovine chromosome BTA9 (according to the positions employed in this analysis) and for bm7234 about 88.136 cM according to the to the MARC marker map. The at least one genetic marker is selected from the group of markers shown in Table 14.

Table 14

| Marker on BTA9 | Position employed in analysis (cM) | Relative position (cM) http://www.marc.usda.gov/ |
| --- | --- | --- |
| EPM2A* | 72.1 | |
| BM7234 | 72.3 | 88.136 |
| * denotes markers that are not listed on the MARC marker map at BTA9. | | |

In one embodiment of the invention the at least one genetic marker is located on the bovine chromosome BTA9. In one embodiment the at least one genetic marker is located on the bovine chromosome BTA9 in the region flanked by and including the markers inra144 and rgs17. In one embodiment of the present invention, the at least one genetic marker is located in the region from about 74.2 cM to about 79.8 cM (according to the positions employed in this analysis) on the bovine chromosome BTA9 where the position of inra144 according to the MARC marker map is 90.98 cM. The at least one genetic marker is selected from the group of markers shown in Table 15.

Table 15

| Marker on BTA9 | Position employed in analysis (cM) | Relative position (cM) http://www.marc.usda.gov/ |
| --- | --- | --- |
| INRA144 | 74.2 | 90.98 |
| INRA084 | 74.5 | 90.98 |

(continued)

| Marker on BTA9 | Position employed in analysis (cM) | Relative position (cM) http://www.marc.usda.gov/ |
| --- | --- | --- |
| rgs17* | 79.8 | - |
| * denotes markers that are not listed on the MARC marker map at BTA9. | | |

In another embodiment of the invention the at least one genetic marker is located on the bovine chromosome BTA9. In one embodiment the at least one genetic marker is located on the bovine chromosome BTA9 in the region flanked by and including the markers inra084 and rgs17. In one embodiment of the present invention, the at least one genetic marker is located in the region from about 74.5 cM to about 79.8 cM (according to the positions employed in this analysis) on the bovine chromosome BTA9 and where the position of inra084 according to the MARC marker map is 90.68 cM. The at least one genetic marker is selected from the group of markers shown in Table16.

Table 16

| Marker on BTA9 | Position employed in analysis (cM) | Relative position (cM) http://www.marc.usda.gov/ |
| --- | --- | --- |
| INRA084 | 74.5 | 90.98 |
| rgs17* | 79.8 | - |
| * denotes markers that are not listed on the MARC marker map at BTA9. | | |

**Primers**

The primers that may be used according to the present invention are shown in Table 17. The in Table 17 specified primer pairs may be used individually or in combination with one or more primer pairs of Table 17.

The design of such primers or probes will be apparent to the molecular biologist of ordinary skill. Such primers are of any convenient length such as up to 50 bases, up to 40 bases, more conveniently up to 30 bases in length, such as for example 8-25 or 8-15 bases in length. In general such primers will comprise base sequences entirely complementary to the corresponding wild type or variant locus in the region. However, if required one or more mismatches may be introduced, provided that the discriminatory power of the oligonucleotide probe is not unduly affected. The primers/probes of the invention may carry one or more labels to facilitate detection.

In one embodiment, the primers and/or probes are capable of hybridizing to and/or amplifying a subsequence hybridizing to a single nucleotide polymorphism containing the sequence delineated by the markers as shown herein.

The primer nucleotide sequences of the invention further include: (a) any nucleotide sequence that hybridizes to a nucleic acid molecule comprising a genetic marker sequence or its complementary sequence or RNA products under stringent conditions, e.g., hybridization to filter-bound DNA in 6x sodium chloride/sodium citrate (SSC) at about 45°C followed by one or more washes in 0.2x SSC/0.1% Sodium Dodecyl Sulfate (SDS) at about 50-65°C, or (b) under highly stringent conditions, e.g., hybridization to filter-bound nucleic acid in 6x SSC at about 45°C followed by one or more washes in 0.1×SSC/0.2% SDS at about 68°C, or under other hybridization conditions which are apparent to those of skill in the art (see, for example, Ausubel F.M. et al., eds., 1989, Current Protocols in Molecular Biology, Vol. I, Green Publishing Associates, Inc., and John Wiley & sons, Inc., New York, at pp. 6.3.1-6.3.6 and 2.10.3). Preferably the nucleic acid molecule that hybridizes to the nucleotide sequence of (a) and (b), above, is one that comprises the complement of a nucleic acid molecule of the genomic DNA comprising the genetic marker sequence or a complementary sequence or RNA product thereof.

Among the nucleic acid molecules of the invention are deoxyoligonucleotides ("oligos") which hybridize under highly stringent or stringent conditions to the nucleic acid molecules described above. In general, for probes between 14 and 70 nucleotides in length the melting temperature (TM) is calculated using the formula:

$$Tm(°C)=81.5+16.6(\log [\text{monovalent cations (molar)}])+0.41(\% \text{ G+C})-(500/N)$$

where N is the length of the probe. If the hybridization is carried out in a solution containing formamide, the melting temperature is calculated using the equation $Tm(°C)=81.5+16.6(\log[\text{monovalent cations (molar)}])+0.41 (\% \text{ G+C})-(0.61 \% \text{ formamide})-(500/N)$ where N is the length of the probe. In general, hybridization is carried out at about 20-25 degrees below Tm (for DNA-DNA hybrids) or 10-15 degrees below Tm (for RNA-DNA hybrids).

Exemplary highly stringent conditions may refer, e.g., to washing in 6x SSC/0.05% sodium pyrophosphate at 37°C (for about 14-base oligos), 48°C (for about 17-base oligos), 55°C (for about 20-base oligos), and 60°C (for about 23-base oligos).

Accordingly, the invention further provides nucleotide primers or probes which detect the polymorphisms of the invention. The assessment may be conducted by means of at least one nucleic acid primer or probe, such as a primer or probe of DNA, RNA or a nucleic acid analogue such as peptide nucleic acid (PNA) or locked nucleic acid (LNA).

According to one aspect of the present invention there is provided an allele-specific oligonucleotide probe capable of detecting a polymorphism at one or more of positions in the delineated regions.

The allele-specific oligonucleotide probe is preferably 5-50 nucleotides, more preferably about 5-35 nucleotides, more preferably about 5-30 nucleotides, more preferably at least 9 nucleotides.

## Determination of linkage

In order to detect if the genetic marker is present in the genetic material, standard methods well known to persons skilled in the art may be applied, e.g. by the use of nucleic acid amplification. In order to determine if the genetic marker is genetically linked to mastitis resistance traits, a permutation test can be applied (Doerge and Churchill, 1996), or the Piepho-method can be applied (Piepho, 2001). The principle of the permutation test is well described by Doerge and Churchill (1996), whereas the Piepho-method is well described by Piepho (2001). Significant linkage in the within family analysis using the regression method, a 10000 permutations were made using the permutation test (Doerge and Churchill, 1996). A threshold at the 5% chromosome wide level was considered to be significant evidence for linkage between the genetic marker and the mastitis resistance and somatic cell count traits. In addition, the QTL was confirmed in different sire families. For the across family analysis and multi-trait analysis with the variance component method, the Piepho-method was used to determine the significance level (Piepho, 2001). A threshold at the 5% chromosome wide level was considered to be significant evidence for linkage between the genetic marker and the mastitis resistance and somatic cell count traits.

## Kit

Another aspect of the present invention relates to use of a diagnostic kit for detecting the presence or absence in a bovine subject of at least one genetic marker associated with resistance to mastitis, comprising at least one oligonucleotide sequence and combinations thereof, wherein the nucleotide sequences are selected from any of SEQ ID NO.: 75 to SEQ ID NO.: 98.

Genotyping of a bovine subject in order to establish the genetic determinants of resistance to mastitis for that subject according to the present invention can be based on the analysis of DNA and/or RNA. One example is genomic DNA which can be provided using standard DNA extraction methods as described herein. The genomic DNA may be isolated and amplified using standard techniques such as the polymerase chain reaction using oligonucleotide primers corresponding (complementary) to the polymorphic marker regions. Additional steps of purifying the DNA prior to amplification reaction may be included. Thus, a diagnostic kit for establishing mastitis resistance and somatic cell count characteristics comprises, in a separate packing, at least one oligonucleotide sequence selected from the group of sequences shown in table 17 and any combinations thereof.

## Examples- BTA9

Animals

The animal material consists of a grand daughter design with 39 paternal sire families with a total number of offspring tested sons was 1513 from four dairy cattle breeds namely Danish Holstein (DH) and Danish Red (DR), Finnish Ayrshire (FA) and Swedish Red and White (SRB). These 39 families consist of 5 DH, 9 DR, 11 FA and 14 SRB grandsire families. The number of sons per grandsire ranged from 16 to 161, with an average family size of 38.8.

Purification of genomic DNA

Genomic DNA was purified from semen according to the following protocol:

After thawing the semen-straw, both ends of the straw were cut away with a pair of scissors and the content of semen transferred to a 1.5 ml eppendorf tube. 1 ml of 0.9% NaCl was used to flush the straw into the tube. The tube was then centrifuged for 5 minutes at 2000 rpm, followed by removal of the supernatant. This washing step was repeated twice.

Then 300 μl buffer S (10 mM Tris HCl pH 8, 100 mM NaCl, 10 mM EDTA pH 8; 0,5 % SDS), 20 μl 1 M DTT and

20 μl pronase (20 mg/ml) (Boehringer )are added to the tube. After mixing the tubes are incubated over night with slow rotation where after 180 μl saturated NaCl is added followed by vigorous agitation for 15 seconds. The tube is the centrifuged for 15 minutes at 11000 rpm. 0.4 ml of the supernatant is transferred to a 2 ml tube and 1 ml of 96% ethanol is added, mixing is achieved by slow rotation of the tube. The tube is then centrifuged for 10 minutes at 11000 rpm. Remove the supernatant by pouring away the liquid, wash the pellet with 70% ethanol (0.2 ml) and centrifuge again for 10 minutes at 11000 rpm. Pour away the ethanol, dry the pellet and resuspend in 0.5 ml of TE-buffer) for 30 minutes at 55°C.

Amplification procedures

PCR reactions were run in a volume of 8 μl using TEMPase (GeneChoice) polymerase and reaction buffer I as provided by the supplier (GeneChoice). Usually 5 different markers are included in each multiplex PCR. 1 μl DNA, 0.1 μl TEMPase enzyme, 0.2 mM dNTPs, 1.2 mM MgCl2, 0.3μM each primer.

The PCR mixtures were subjected to initial denaturation at 94°C for 15 min (for TEMPase). Subsequently, the samples were cycled for 10 cycles with touchdown, i.e. the temperature is lowered 1°C at each cycle (denaturation at 94°C 30°, annealing at 67°C 45", elongation 72°C 30"), after which the samples were cycled for 20 cycles with normal PCR conditions (denaturation at 94°C 30", annealing at 58°C 45", elongation 72°C 30) PCR cycling was terminated by 1 cycle at 72°C 30' and the PCR machine was programmed to cooling down the samples at 4°C for 'ever'.

The nucleotide sequence of the primers used for detecting the markers is shown in Table 17. The nucleotide sequence is listed from the 5' end.

Table 17

| Marker name | Forward Primer F | SEQ ID NO.: |
|---|---|---|
| | Reverse Primer R | |
| **BTA9:**<br>C6orf93 | F CTCGGTGATGTTTTTGCTGA<br>R CGCCCCAGCTCTTTCTAGTT | SEQ ID NO.: 75<br>SEQ ID NO.: 76 |
| DIK 4986 | F GGGATGAACATTGAGGGTTG<br>R CATGATCAAGATGGGGGAAG | SEQ ID NO.: 77<br>SEQ ID NO.: 78 |
| Mm12e6 | F CAAGACAGGTGTTTCAATCT<br>R ATCGACTCTGGGGGATGATGT | SEQ ID NO.: 79<br>SEQ ID NO.: 80 |
| PEX3 | F TTTTGCGAGTCCAGTTAAACA<br>R GGAAAAGCCAGAGCAAAATG | SEQ ID NO.: 81<br>SEQ ID NO.: 82 |
| DEADC1 | **F** TTCCTAGGCCTGTGCTCATT<br>R TGGACCAGGCATAAGGATTT | SEQ ID NO.: 83<br>SEQ ID NO.: 84 |
| BMS2251 | F AACGGCTTTCACTTTCTTGC<br>R CTGGGTGAACAAATGGGC | SEQ ID NO.: 85<br>SEQ ID NO.: 86 |
| EPM2A | F GCGGCC GCGTTGAGAG<br>R TTCCAC TTT ATG ATG AGC AGG TTC | SEQ ID NO.: 87<br>SEQ ID NO.: 88 |
| BM7234 | F TTCACTGATTGTCATTCCCTAGA<br>R TAAGCAAATAAATGGTGCTAGTCA | SEQ ID NO.: 89<br>SEQ ID NO.: 90 |
| BM4208 | F TCAGTACACTGGCCACCATG<br>R CACTGCATGCTTTTCCAAAC | SEQ ID NO.: 91<br>SEQ ID NO.: 92 |
| BMS2819 | F GCTCACAGGTTCTGAGGACTC<br>R AACTTGAAGAAGGAATGCTGAG | SEQ ID NO.: 93<br>SEQ ID NO.: 94 |
| INRA144 | **F** TCGGTGTGGGAGGTGACTACAT<br>R TGCTGGTGGGCTCCGTCACC | SEQ ID NO.: 95<br>SEQ ID NO.: 96 |
| INRA084 | F CTAAAGCTTTCCTCCATCTC<br>R CCTGGTGATGTTTGGATGTC | SEQ ID NO.: 97<br>SEQ ID NO.: 98 |

Markers and Map

For BTA9 in the present study 45 microsatellite markers were chosen from the web-site of the Meat Animal Research Center (*www.marc.usda.gov/genome/genome.html*). As BTA9 is orthologous to HSA6q, 28 published genes and ESTs were chosen along HSA6q (*Ctgf, Vip, Vil2, Rgs17, Ros1, Slc16a10, Oprm, igf2r, Esr1, Deadc1, Pex3, C6orf93, Ifngr1, Shprh, Epm2a, AK094944, AK094379, Utm, Tnf, plg, arid1b, lama4, hivep2, C6orf055, CITED2, RP1-172K10, AIG1, GRM*) for SNPs and microsatellites screening. Eight of new microsatellite markers identified in the present study and 29 SNPs were also geno-typed across the pedigree in order to create a dense map of BTA9 by linkage analysis.

Radiation hybrid (RH) panel information

Specific primer pairs were designed from the bovine sequences to map the genes and microsatellites including MARC microsatellites. Along chromosome 9, a total of more than 120 markers were used on the cattle RH panel. 65 genes and 34 microsatellites showed a successful amplification, bands with the appropriate size on the bovine DNA and no amplification on hamster DNA. They were typed on the 3000-rad panel Rostin/Cambridge bovine RH panel (Williams et al. 2002).

PCR amplifications were performed in a total volume of 20 $\mu$l containing 25 ng of the RH cell line DNA, 0.5 $\mu$M of each primer, 200 $\mu$M dNTPs, 3 mM MgCl$_2$, 0.5U of Taq polymerase (BIOLine). The reaction conditions were a touch-down starting with 94°C for 3 min followed by 40 cycles of 93°C for 30s, 65-45°C touch-down for 30 s, decreasing 0.5°C per cycle, and 72°C for 1 min, with a final extension step of 72°C for 5 min. PCR reactions were electrophoresed: 10 $\mu$l of the PCR product were loaded in ethidium bromide stained mini-gels (2.5 % agarose) and the presence or absence of amplicons were scored by two independent observers. Where there were several discrepancies between the patterns from the duplicates or between the scores from the different observers, PCR reactions and gels were repeated. Markers were discarded when the results for several hybrids remained ambiguous.

Markers were assigned to the bovine chromosome by canying out 2-point linkage analysis using RHMAPPER (Soderlund et al., 1998) against markers with known assignments that had been previously typed on the bovine WGRH panel (Williams et al., 2002). RH map was then constructed using the Carthegene software (Schiex., 2002) as described by Williams et al (2002). On bovine chromosome 9, we have information from a radiation hybrid map with 150 markers.

Marker order and map distances were estimated using CRIMAP 2.4 software (Green et al. 1990). To construct our linkage map we began by placing the microsatellite markers following the MARC map order. Next a BUILD option of CRIMAP was run to place the remaining markers, the new microsatellite and SNP markers have been inserted at the position with the highest likelihood. MARC (*www.marc.usda.gov/genome/genome.html*), Ensembl (http://www.ensembl.org/Bos_taurus/index.html) and radiation hybrid (RH) information have been taken into account to reconsider that emplacement of the makers. The final linkage map used of the QTL mapping of BTA9 according to the present invention includes 59 markers as listed in table 18.

The following table show markers used for the relevant QTL. Any additional information on the markers can be found on 'http://www.marc.usda.gov/', http://www.ensembl.org/Bos_taurus/index.html and 'http://www.ncbi.nih.gov/'.

Table 18

| Marker on BTA9 | Position employed in analysis (cM) | Relative position (cM) http://www.marc.usda.gov/ |
| --- | --- | --- |
| BMS2151 | 0 | 4.892 |
| ETH225 | 7.4 | 12.754 |
| BM2504 | 25.3 | 30.92 |
| DIK2892 | 25.35 | 30.92 |
| DIK 3002 | 32.7 | 36.542 |
| DIK 3003 | 32.75 | 36.542 |
| RM216 | 32.8 | 37.087 |
| BMS817 | 36.6 | 42.489 |
| BMS555 | 37.4 | 43.818 |
| lama4* | 37.6 | - |
| DIK 5142 | 37.75 | 43.818 |
| slc16a10* | 37.78 | - |

(continued)

| Marker on BTA9 | Position employed in analysis (cM) | Relative position (cM) http://www.marc.usda.gov/ |
|---|---|---|
| DIK 4268 | 37.81 | 45.152 |
| DIK 4950 | 37.84 | 45.152 |
| CSSM025 | 37.87 | 45.739 |
| DIK 2810 | 37.9 | 45.739 |
| DIK 5364 | 38.2 | 45.739 |
| DIK 2741 | 39.75 | 49.659 |
| TGLA261 | 39.8 | 49.659 |
| ILSTS013* | 39.85 | - |
| UWCA9 | 39.9 | 49.996 |
| BMS1148 | 39.95 | 50.923 |
| DIK 4912 | 42.45 | 51.855 |
| DIK 5130 | 42.5 | 52.296 |
| DIK 2303 | 42.55 | 52.352 |
| DIK 4720 | 43.3 | 53.966 |
| BM4204 | 45.1 | 55.414 |
| DIK 4926 | 47.6 | 57.088 |
| BMS1909 | 49.8 | 59.516 |
| BMS1290 | 53.8 | 64.935 |
| TGLA73 | 63.3 | 77.554 |
| BMS2753 | 65.4 | 79.249 |
| TNF* | 65.45 | - |
| BMS1724 | 67.15 | 80.265 |
| DIK 2145 | 67.2 | 80.265 |
| BM7209 | 67.6 | 81.569 |
| SLU2 | 68.8 | - |
| C6orf93* | 69.35 | - |
| DIK 4986 | 69.4 | 84.258 |
| mm12e6* | 69.45 | 84.258 |
| PEX3* | 69.5 | - |
| DEADC1 | 69.55 | - |
| BMS2251 | 71.3 | 86.58 |
| EPM2A* | 72.1 | |
| BM7234 | 72.3 | 88.136 |
| BM4208 | 73.9 | 90.69 |
| BMS2819 | 73.95 | 90.98 |
| INRA144 | 74.2 | 90.98 |
| INRA084 | 74.5 | 90.98 |
| rgs17* | 79.8 | - |

(continued)

| Marker on BTA9 | Position employed in analysis (cM) | Relative position (cM) http://www.marc.usda.gov/ |
|---|---|---|
| ESR1* | 79.85 | - |
| BMS2295 | 82.2 | 98.646 |
| BM3215 | 83.2 | 101.647 |
| bvil203* | 86.5 | - |
| Aridlb* | 86.6 | - |
| Plg* | 89.4 | - |
| igfsnp123* | 89.45 | - |
| BMS1943 | 92.5 | 103.708 |
| BMS1967 | 97.7 | 109.287 |
| * these markers are not listed in the MARC marker map of BTA9. | | |

Phenotypic Data

Daughters of bulls were scored for mastitis resistance and SCS. Estimated breeding values (EBV) for traits of sons were calculated using a single trait Best Linear Unbiased Prediction (BLUP) animal model ignoring family structure. These EBVs were used in the QTL analysis. The daughter registrations used in the individual traits were:

Clinical mastitis in Denmark: Treated cases of clinical mastitis in the period -5 to 50 days after 1st calving.
Clinical mastitis in Sweden and Finland: Treated cases of clinical mastitis in the period -7 to 150 days after 1st calving.
SCS in Denmark: Mean SCS in period 10-180 days after 1st calving.
SCS in Sweden: Mean SCS in period 10-150 days after 1st calving.
SCS in Finland: Mean SCS in period 10-305 days after 1st calving.

Statistical analysis

A number of statistical methods as described below were used in the determination of genetic markers associated or linked to mastitis and thus mastitis resistance.

QTL Analysis

Linkage analysis (LA) is used to identify QTL by typing genetic markers in families to chromosome regions that are associated with disease or trait values within pedigrees more often than are expected by chance. Such linked regions are more likely to contain a causal genetic variant. The data was analysed with a series of models. Initially, a single trait model using a multipoint regression approach for all traits were analysed within family. Chromosomes with significant effects within families were analysed with the variance component method to validate QTL found across families and for characterization of QTL.

Regression analysis

Population allele frequencies at the markers were estimated using an EM-algorithm. Allele frequencies were subsequently assumed known without error. Phase in the sires was determined based on offspring marker types. Subsequently this phase was assumed known without error. Segregation probabilities at each map position were calculated using information from all markers on the chromosome simultaneously using Haldane's mapping function (Haldane, 1919). Phenotypes were regressed onto the segregation probabilities. Significance thresholds were calculated using permutation tests (Churchill and Doerge, 1994).

Variance component method

The across-family linkage analysis was carried out using variance component (VC) based method (Sørensen et al., 2003). In LA with VC, the Identity by descent (IBD) probabilities between QTL alleles of any two founder haplotypes (Hs and Hm) are assumed to be zero, i.e. founder haplotypes were unrelated (Meuwissen et al. 2002). The sire haplotypes and the paternally inherited haplotypes of the sons are used to compute the probability of inheriting the paternal or maternal QTL allele from the sire (Freyer et al. 2004) and computed the IBD matrix using a recursive algorithm (Wang et al., 1995). The IBD matrices were computed at the midpoint of each marker bracket along the chromosome and used in the subsequent variance component estimation procedure. The fraction of the total additive genetic variance explained by the QTL was estimated as $2\sigma^2_h / (2\sigma^2_h + \sigma^2_u)$ where $\sigma^2_h$ and $\sigma^2_u$ correspond respectively to the variance component associated with the haplotypes effect and the additive polygenic effect.

Variance component analysis. Single trait single QTL analysis.

Each trait was analysed separately using linkage analysis. The full model can be expressed as:

$$y = X\beta + Zu + Wq + e, \tag{1}$$

where y is a vector of n EBVs, X is a known design matrix, $\beta$ is a vector of unknown fixed effects, which is in this case only the mean, Z is a matrix relating to individuals, u is a vector of additive polygenic effects, W is a known matrix relating each individual record to its unknown additive QTL effect, q is a vector of unknown additive QTL effects of individuals and e is a vector of residuals. The random variables u, q and e are assumed to be multivariate normally distributed and mutually independent (Lund et al., 2003).

Multi-trait multi-QTL analysis

Multi-trait analysis was performed. Model (1) can be extended to a multi-trait multi-QTL model as described in Model (2) following Lund et al., 2003.

The traits are modeled using the following linear mixed model with $n_q$ QTL:

$$y = \mu + Za + \sum_{i=1}^{n_q} Wh_i + e, \tag{2}$$

where y is a vector of observations for n sons recorded on t traits, $\mu$ is a vector of overall trait means, Z and W is known matrices associating the observations of each son to its polygenic and QTL effects, a is a vector of polygenic effects of sires and their sons, $h_i$ is a vector of QTL haplotypes effects of sires and their sons for the i'th QTL and e is a vector of residuals. The random variables a, $h_i$ and e are assumed to be multivariate normally distributed (MVN) and mutually uncorrelated. Specifically, a is MVN $(0, G \otimes A)$, $h_i$ is MVN $(0, K_i \otimes IBD_i)$ and e is MVN $(0, E \otimes I)$. Matrices G, K and E include variances and covariances among the traits due to polygenic effects, QTL effects and residuals effects. The symbol $\otimes$ represents the Kronecker product. A is the additive relationship matrix that describe the covariance structure among the polygenic effects, $IBD_i$ is the identity by descent (IBD) matrix that describes the covariance structure among the effects for the i'th QTL, and I is the identity matrix.

Combined linkage and linkage disequilibrium analysis

In combined linkage and linkage disequilibrium analysis, the IBD probabilities between QTL alleles of any two founder haplotypes were computed using the method described by Meuwissen and Goddard (2001). This method approximates the probability that the two haplotypes are IBD at a putative QTL conditional on the identity-by-state (IBS) status of flanking markers, on the basis of coalescence theory (Hudson, 1985). Briefly, the IBD probability at the QTL is based on the similarity of the marker haplotypes surrounding alleles that surround the position: i.e. many (non) identical marker alleles near the position imply high (low) IBD probability at the map position. The actual level of IBD probabilities is affected by the effective population size, Ne. The probability of coalescence between the current and an arbitrary base generation, Tg generations ago is calculated given the marker alleles that both haplotypes have in common (Hudson, 1985). It is not easy to estimate Tg and Ne from the observed data. Simulation studies show that the estimate of QTL position is relatively insensitive to choice of Ne and Tg (Meuwissen and Goddard, 2000). Therefore we used the values of Tg = 100 and Ne = 100. Windows of 10 markers were considered to compute the IBD probabilities. We also used 4-markers window to compute IBD probabilities at the area of LDLA peak to examine if 4 markers were sufficient to reproduce the peak already identified by 10-marker haplotypes. Founder haplotypes were grouped into functionally distinct clusters. We used $(1-IBD_{ij})$ as a distance measure and applied the hierarchical clustering algorithm average linkage to generate a rooted dendrogram representing the genetic relationship between all founder haplotypes. The tree is scanned downward from the root and branches are cut until nodes are reached such that all coalescing haplotypes have a distance measure (1-IBD$_{ij}$) < Tc. A cluster is defined as a group of haplotypes that coalesce into a common node. Haplotypes within a cluster are assumed to carry identical QTL allele (IBD probability=1.0) whereas haplotypes from different clusters carry distinct QTL alleles and are therefore considered to be independent (IBD probability=0). Therefore the upper part of the IBD matrix corresponding to the linkage disequilibrium information is an identity matrix corresponding to the distinct founder haplotypes. The lower part of the IBD matrix corresponding to the linkage information in the paternal haplotypes of the sons is build using a recursive algorithm (Wang et al., 1995). The IBD matrices were computed at the midpoints of each marker interval and used in the subsequent variance component estimation procedure.

Estimation of parameters

The variance components were estimated using the average information restricted maximum likelihood algorithm (Jensen et al., 1997). The restricted likelihood was maximized with respect to the variance components associated with the random effects in the model. Maximizing a sequence of restricted likelihoods over a grid of specific positions yields a profile of the restricted likelihood of the QTL position (Sørensen et al., 2003). The parameters were estimated at the mid point of each marker bracket along the chromosome.

Significance level

Significance thresholds for the variance-component analyses were calculated using a quick method to compute approximate threshold levels that control the genome-wise type I error (Piepho, 2001). Hypothesis tests for the presence of QTL were based on the asymptotic distribution of the likelihood ratio test (LRT) statistic, LRT = -2ln(L$_{reduced}$ - L$_{full}$), where L$_{reduced}$ and L$_{full}$ were the maximized likelihoods under the reduced model and full model, respectively. The reduced model always excluded the QTL effect for the chromosome being analyzed. This method is an alternative to permutation procedures and is applicable in complex situations. It requires the LRT from each of the putative QTL positions along the chromosome, the number of chromosomes, the degrees of freedom (df) for the LRT (df = number of parameters of H$_{full}$ - number of parameters of H$_{reduced}$), and the chromosome-wise type I error rate. A significance level of 5% chromosome wise was considered to be significant.

Results BTA9

In table 19 the results from the regression analysis for BTA9 are presented. Figures 1 to 8 present the QTL graphs for the regression analysis. The variance component method was used to detect QTL across families QTL analysis. Figures 9 to 16 present the LD, LDLA and LD profile for the QTL in a variance component based method. Figures 17 to 20 present the haplotypes effects.

Danish Red

Within family regression analysis revealed that QTL for CM and SCS are segregating in two families in DR breed. The QTL for two traits were not located in the same interval. In across-family linkage analysis using VC method, the QTL effects were not significant. With LDLA and LD analyses, high QTL peaks were observed at 74.08 cM between markers BMS2819 and INRA144. The peak LRT in LD analysis (13.6) was higher than the peak LRT (8.51) observed in combined LDLA analysis. This QTL explained 44% and 22% of the additive genetic variance and phenotypic variance for CM respectively. By default 10 marker haplotypes (five markers on each side of the putative position) were used to estimate the IBD probability of a location. We also used 4 marker haplotypes i.e. 2 markers on each side of the putative position, and observed similar LDLA/LD peak within these four marker (BM4208-BMS2819-INRA144-INRA084) haplotypes. A LDLA combined peak for SCS was also observed within these 4 markers bracket in this breed.
The dam haplotypes with IBD probability of 0.90 or above were clustered together. There were 305 founder haplotypes in DR before clustering which reduced to 54 clusters after clustering. Five clusters had frequency higher than 5% and the largest cluster had a frequency of 10% and five sire haplotypes were also clustered with the largest cluster. The haplotypes effects for these 54 haplotypes and also the haplotypes received from the sires were estimated. The haplotypes associated with high and low mastitis resistance were identified.

Finnish Ayrshire

The QTL affecting CM and SCS were found segregating when within family regression analysis was performed in Finish Ayrshire families. The QTL for CM was located in the interval of 58 to 79 cM. The QTL affecting SCS was located in between 32 to 44 cM with the peak LRT statistics at 37 cM. The combined LDLA peak for CM QTL over LA profile was observed within the markers BM4208-BMS2819-INRA144. The LD peak was also observed in the same region for CM. One LDLA peak for SCs over LA profile was observed at 38 cM between the markers DIK2810 and DIK5364. Four percent of the total variance in CM was explained by the QTL at 74 cM and this QTL showed no effect on SCS in FA. The QTL at 38 cM explained 18% of the total variance in SCS and it had very small effect on CM. At the highest LDLA peak in CM i.e. in the mid interval between markers BM4208 and BMS2819, 442 founder haplotypes grouped into 38 clusters when the clustering probability of 0.90 was applied. There were nine clusters with frequency higher than 5%. The biggest cluster had a frequency of 14%. The haplotypes associated with high and low mastitis resistance were identified.

Swedish Red And White

Similar to DR and FA cattle, QTL affecting CM and SCS were also observed segregating on BTA9 in Swedish Red and White cattle when within-family regression analyses were performed. Both the QTL for CM and SCS were located in the same interval. The CM QTL was significant (P<0.01) in across-family LA analysis. The SCS QTL was not significant in across-family LA analysis. The peak LRT for SCS was at 73cM. The peak test statistics for CM QTL in across-family analysis was at 67.4 cM with the QTL interval was between 59 and 81 cM. Though the LRT statistics was highly significant in across-family LA analysis, no LDLA peak over LA profile was observed for this QTL in SRB cattle. At the peak LRT statistics location in LA analysis, the QTL variance was 25% of the total variance of CM trait. LDLA peaks for CM QTL of DR and FA breeds fall within the LA profile observed in SRB. Though no LDLA peak was observed in SRB data for CM, there were lot of clustering in SRB in the marker intervals where the peaks in DR and FA was located. For example at the mid interval between BM4208-BMS2819, where the highest LDLA peak is located in FA and in the neighbouring interval the DR peak (BMS2819-INRA144), there were 37 and 48 clusters respectively, out of 400 total founder haplotypes in SRB.

Danish Holstein

QTL affecting CM and SCS was also segregating in Danish Holstein cattle revealed in within-family regression analysis. The CM QTL was significant (P<0.01) in across-family LA analysis with VC method. Though small LDLA peaks over LA profile was observed, but no convincing LD peak was seen for the QTL in DH. The highest LRT statistics for CM QTL in LA was at 42.9 cM with the LRT statistics of 10.6 and the QTL interval was quite large spreading from 29 to 51 cM. One small LD peak for CM QTL coincides with the LD peaks observed in DR and FA population at 74 cM. The SCC QTL has peak test statistics at 48.7 cM with an interval from 44 to 58 cM. The part of total variance explained by the QTL taking the highest peaks in respective LA were 27 and 17% for CM and SCS respectively. The highest LD peak for CM was at 73.35cM, the region where high LD peak for DR was observed. No LD peak for SCS was observed in DH.

Across breed analysis

Within-breed LA, LDLA and LD analyses revealed that the QTL affecting CM were segregating at around 74 cM in more than one population. Therefore, across-breed QTL analyses were carried out combing data across different breeds in the study. The results of across-breed QTL analysis are presented in Tables 20, 21 and 22. The LDLA peak for CM QTL in DR and FA cattle was located in the neighbouring marker intervals when within breed analyses were done. However, a high LDLA peak of CM was observed in the marker bracket (BMS2819-INRA144) when combined data of DR and FA were analyzed and also coincides with the LD peak. The combined analysis of FA and SRB data didn't gave any higher LDLA peak over LA profile, however, the LD peak was observed at the same marker interval at 74 cM. The analyses of combined DR, FA and SRB data also gave the higher LDLA peak over LA in the same region i.e. BM4208-BMS2819-INRA144. The LD peak was also at the same location which authenticated the higher LDLA peak over LA.

The joint analysis of DR and FA showed a high LDLA peak at 38 cM between the markers DIK2810 and DIK5364 for SCS QTL. LDLA peak at the same location was also observed for SCS QTL in combined analysis of FA and SRB. However, this LDLA peak disappeared when DR, FA and SRB were analyzed together.

Multi-trait analysis

SCS is an indicator trait of mastitis resistance. It was expected that many of genes responsible of CM will also have effect on SCS. Therefore, multi-trait analysis of CM and SCS was carried out to test if the QTL segregating on BTA9 have pleiotropic effect on both the traits or they are linked QTL. Though the single-trait LDLA analysis of DR data showed the LDLA peak of CM and SCS at the same marker interval i.e. between BMS2819 and INRA144, the combined analysis of 2-traits gave LDLA peak at 69.1 cM in between the markers SLU2 and C6orf93. In within-breed analysis FA, SR did not show LDLA peak for the model with QTL affecting both CM and SCS. However when the three breeds. DR, FA and SRB were combined and analyzed with a 2-trait model, LDLA peak with LRT statistics of 19.7 was observed in the marker interval INRA144 and INRA084.

Haplotype analysis

QTL fine mapping results mentioned above, points towards a QTL segregating for CM within the 4-marker region, BM4208-BMS2819-INRA144-INRA084. Therefore the clustering of founder haplotypes and haplotypes effects were studied at the midpoint between the markers BMS2819 and INRA144. This was done within breeds and also across three breeds DR, FA and SRB as these three breeds are related in their origin. The haplotypes associated with high and low mastitis resistance were identified.

Table 19.

| With-family linkage analysis using regression interval analysis. | | | | | | |
|---|---|---|---|---|---|---|
| Breed and Sire No. | Mastitis resistance (CM) | | | Somatic Cell Count | | |
| | Position (Morgan) | F-values | p-values | Position (Morgan) | F-values | p-values |
| **Danish Holstein** | | | | | | |
| 1079 | 0.829 | 10.73 | 0.99 | 0.596 | 13.19 | 1.00 |
| 1080 | 0.432 | 3.79 | 0.73 | 0.072 | 0.91 | 0.12 |
| 1082 | 0.643 | 6.76 | 0.92 | 0.654 | 1.18 | 0.21 |
| 1087 | 0.474 | 4.22 | 0.76 | 0.919 | 7.58 | 0.95 |
| 1808 | 0.347 | 16.61 | 1.00 | 0.596 | 1.97 | 0.33 |
| **Across-family** | **0.432** | **4.90** | **1.00** | **0.523** | **3.43** | **0.97** |
| **Danish Red** | | | | | | |
| 1800 | 0.501 | 13.00 | 0.99 | 0.501 | 5.65 | 0.86 |
| 1801 | 0.728 | 1.06 | 0.57 | 0.728 | 0.36 | 0.28 |
| 1802 | 0.699 | 0.07 | 0.07 | 0.961 | 0.23 | 0.21 |
| 1803 | 1.009 | 1.75 | 0.37 | 0.156 | 11.35 | 0.99 |
| 1804 | 0.718 | 0.32 | 0.18 | 0.712 | 3.96 | 0.88 |
| 1806 | 0.739 | 0.07 | 0.05 | 0.654 | 1.30 | 0.60 |
| 1807 | 0.363 | 0.28 | 0.24 | 0.442 | 0.45 | 0.34 |
| 4009 | 0.696 | 2.46 | 0.86 | 0.358 | 0.73 | 0.59 |
| **Across-family** | 0.502 | 1.95 | 0.81 | 0.497 | 1.77 | 0.71 |
| **Finnish Ayrshire** | | | | | | |
| 34872 | 0.913 | 0.83 | 0.51 | 0.416 | 1.17 | 0.62 |
| 35142 | 0.363 | 0.44 | 0.26 | 0.358 | 0.55 | 0.32 |
| 36386 | 1.003 | 3.14 | 0.71 | 1.003 | 1.95 | 0.50 |
| 36455 | 0.617 | 0.96 | 0.49 | 0.903 | 1.76 | 0.69 |
| 36460 | 0.792 | 8.81 | 0.97 | 0.945 | 8.49 | 0.97 |
| 36687 | 0.718 | 8.27 | 0.95 | 0.564 | 3.11 | 0.63 |
| 36733 | 0.728 | 1.67 | 0.74 | 0.728 | 0.66 | 0.49 |

(continued)

| Finnish Ayrshire | | | | | | |
|---|---|---|---|---|---|---|
| 37465 | 0.538 | 3.24 | 0.71 | 0.368 | 15.85 | 1.00 |
| 37505 | 0.358 | 1.31 | 0.58 | 0.358 | 1.66 | 0.66 |
| 38393 | 0.358 | 0.64 | 0.55 | 0.384 | 1.99 | 0.80 |
| 38651 | 0.808 | 1.33 | 0.56 | 0.998 | 2.17 | 0.72 |
| **Across-family** | **0.687** | **2.03** | **0.90** | **0.978** | **1.94** | **0.89** |
| **Swedish Red** | | | | | | |
| 36460 | 0.792 | 5.22 | 0.85 | 0.945 | 9.92 | 0.97 |
| 74746 | 0.426 | 5.89 | 0.91 | 0.638 | 2.38 | 0.58 |
| 75241 | 0.702 | 0.10 | 0.06 | 0.744 | 9.47 | 0.99 |
| 76351 | 0.913 | 4.13 | 0.78 | 0.336 | 5.74 | 0.88 |
| 76360 | 0.654 | 1.07 | 0.48 | 0.686 | 1.57 | 0.61 |
| 83798 | 0.834 | 0.09 | 0.09 | 0.670 | 1.17 | 0.59 |
| 85409 | 0.686 | 2.00 | 0.73 | 0.739 | 1.10 | 0.55 |
| 85439 | 0.363 | 3.62 | 0.74 | 0.919 | 1.38 | 0.33 |
| 85679 | 0.903 | 10.82 | 0.97 | 0.336 | 0.32 | 0.01 |
| 85716 | 0.739 | 11.77 | 0.98 | 0.649 | 2.21 | 0.49 |
| 86063 | 0.723 | 4.18 | 0.79 | 0.718 | 1.67 | 0.42 |
| 86097 | 0.432 | 1.68 | 0.53 | 0.760 | 1.51 | 0.50 |
| 86626 | 0.416 | 5.19 | 0.84 | 1.009 | 5.72 | 0.87 |
| **Across-family** | **0.674** | **3.18** | **1.00** | **0.724** | **2.22** | **0.94** |

Table 20.

| Summary of across-family linkage analysis (LA) using variance component method | | | | |
|---|---|---|---|---|
| **Breed** | **Trait** | **Position (Morgan)** | **Peak LRT statistics** | **Marker interval** |
| Danish Red (DR) | CM | 0.464 | 4.43 | BM4208 - DIK4926 |
| | SCS | 0.253 | 4.58 | BMS2504 - DIK2892 |
| Finish Ayrshire (FA) | CM | 0.682 | 4.19 | BM7209 - SLU2 |
| | SCS | 0.370 | 5.12 | BMS817 - BMS555 |
| Swedish Red (SR) | CM | 0.674 | 9.89 | DIK2145 - BM7209 |
| | SCS | 0.731 | 6.05 | BM7234 - BM4208 |
| Danish Holstein (DH) | CM | 0.429 | 10.63 | DIK2303 - DIK4720 |
| | SCS | 0.487 | 6.72 | DIK4926 - BMS1909 |
| DR + FA | CM | 0.682 | 3.20 | BM7209 - SLU2 |
| | SCS | 0.370 | 6.57 | BMS817 - BMS555 |
| FA + SR | CM | 0.682 | 15.30 | BM7209 - SLU2 |
| | SCS | 0.951 | 9.91 | BMS1943 - BMS1967 |

(continued)

| Summary of across-family linkage analysis (LA) using variance component method | | | | |
|---|---|---|---|---|
| **Breed** | **Trait** | **Position (Morgan)** | **Peak LRT statistics** | **Marker interval** |
| DR + FA + SR | CM | 0.682 | 13.53 | BM7209 - SLU2 |
| | SCS | 0.951 | 8.51 | BMS1943 - BMS1967 |

Table 21.

| Summary of linkage disequilibrium and linkage analysis (LDLA) using variance component method | | | | |
|---|---|---|---|---|
| **Breed** | **Trait** | **Position (Morgan)** | **Peak LRT statistics** | **Marker interval** |
| Danish Red (DR) | CM | 0.741 | 8.51 | BM2819 - INRA144 |
| | SCS | 0.398 | 16.95 | DIK2741 - TGLA261 |
| Finish Ayrshire (FA) | CM | 0.739 | 5.38 | BM4208 - BMS2819 |
| | SCS | 0.381 | 7.26 | DIK2810 - DIK5364 |
| Swedish Red (SR) | CM | 0.672 | 5.56 | BMS1724 - DIK2145 |
| | SCS | 0.741 | 5.71 | BMS2819 - INRA144 |
| Danish Holstein (DH) | CM | 0.429 | 12.69 | DIK2303 - DIK4720 |
| | SCS | 0.464 | 7.85 | BM4204 - DIK4926 |
| DR + FA | CM | 0.741 | 9.93 | BMS2819-INRA144 |
| | SCS | 0.381 | 9.61 | DIK2810 - DIK5364 |
| FA + SR | CM | 0.739 | 10.98 | BM4208 - BMS2819 |
| | SCS | 0.691 | 6.26 | SLU2 - C6orf93 |
| DR + FA + SR | CM | 0.739 | 14.90 | BM4208-BMS2819 |
| | SCS | 0.741 | 6.69 | BMS2819 - INRA144 |

Table 22

| Summary of across-family linkage disequilibrium (LD) analysis using variance component method | | | | |
|---|---|---|---|---|
| **Breed** | **Trait** | **Position (Morgan)** | **Peak LRT statistics** | **Marker interval** |
| Danish Red (DR) | CM | 0.741 | 13.60 | BM2819 - INRA144 |
| | SCS | 0.398 | 12.95 | DIK2741 - TGLA261 |
| Finish Ayrshire (FA) | CM | 0.739 | 3.64 | BM4208 - BMS2819 |
| | SCS | - | <1.0 | - |
| Swedish Red (SR) | CM | 0.327 | 4.26 | DIK3002 - DIK3003 |
| | SCS | 0.464 | 2.18 | BM4204 - DIK4926 |
| Danish Holstein (DH) | CM | 0.744 | 5.45 | INRA144 - INRA084 |
| | SCS | 0.731 | 1.49 | BM7234 - BM4208 |
| DR + FA | CM | 0.741 | 5.49 | BMS2819 - INRA144 |
| | SCS | 0.398 | 1.99 | TGLA261 - ILSTS013 |
| FA + SR | CM | 0.739 | 5.39 | BM4208 - BMS2819 |
| | SCS | 0.370 | 1.73 | BMS817 - BMS555 |

(continued)

| Summary of across-family linkage disequilibrium (LD) analysis using variance component method | | | | |
|---|---|---|---|---|
| **Breed** | **Trait** | **Position (Morgan)** | **Peak LRT statistics** | **Marker interval** |
| DR+FA+SR | CM | 0.739 | 8.94 | BM4208 - BMS2819 |
| | SCS | 0.253 | 4.08 | BMS2504 - DIK2892 |

**Claims**

1. A method for determining the resistance to mastitis in any bovine subjects of Danish Red, Ayrshire, Swedish Red and White, and Holstein Breeds, comprising detecting in a sample from said bovine subject the presence or absence of at least one genetic marker that is linked to at least one trait indicative of mastitis resistance, wherein said at least one genetic marker is located on the bovine chromosome BTA9 in the region flanked by and including the polymorphic microsatellite markers C6orf93 and inra084, wherein the presence or absence of said at least one genetic marker is indicative of mastitis resistance of said bovine subject or off-spring therefrom.

2. The method according to claim 1, wherein the at least one genetic marker is located in the region flanked by and including the genetic markers bms2251 and inra084 of BTA9.

3. The method according to claim 1, wherein the at least one genetic marker is located in the region flanked by and including the genetic markers bm7234 and inra144 of BTA9.

4. The method according to claim 1, wherein the at least one genetic marker is located in the region flanked by and including the genetic markers bms2819 and inra144 of BTA9.

5. The method according to claim 1, wherein the at least one genetic marker is located in the region flanked by and including the genetic markers bm4208 and inra144.

6. The method according to claim 1, wherein the at least one genetic marker is the microsatellite marker C6orf93.

7. The method according to claim 1, wherein the at least one genetic marker is the microsatellite marker DIK4986.

8. The method according to claim 1, wherein the at least one genetic marker is the microsatellite marker mm12e6.

9. The method according to claim 1, wherein the at least one genetic marker is the microsatellite marker PEX3.

10. The method according to claim 1, wherein the at least one genetic marker is the microsatellite marker DEAD21.

11. The method according to claim 1, wherein the at least one genetic marker is the microsatellite marker BMS 2251.

12. The method according to claim 1, wherein the at least one genetic marker is the microsatellite marker EPM2A.

13. The method according to claim 1, wherein the at least one genetic marker is the microsatellite marker BM7234.

14. The method according to claim 1, wherein the at least one genetic marker is the microsatellite marker BM4208.

15. The method according to claim 1, wherein the at least one genetic marker is the microsatellite marker BMS2819.

16. The method according to claim 1, wherein the at least one genetic marker is the microsatellite marker INRA144.

17. The method according to claim 1, wherein the at least one genetic marker is the microsatellite marker INRA084.

18. The method according to claim 1, wherein the at least one genetic marker is a combination of genetic markers.

19. The method according to claim 6, wherein the primer pair for amplifying the microsatellite marker C6orf93 is SEQ ID NO.: 75 and SEQ ID NO.:76.

**20.** The method according to claim 9, wherein the primer pair for amplifying the microsatellite marker PEX3 is SEQ ID NO.: 81 and SEQ ID NO.:82.

**21.** The method according to claim 12, wherein the primer pair for amplifying the microsatellite marker EPM2A is SEQ ID NO.: 87 and SEQ ID NO.:88.

**22.** The method according to claim 1, wherein the at least one genetic marker is a combination of genetic markers.

**23.** A method for selecting bovine subjects for breeding purposes, said method comprising by the method in claim 1 determining resistance to mastitis.

**24.** Use of a diagnostic kit comprising at least one oligonucleotide sequence , wherein the nucleotide sequences are selected from any of SEQ ID NO.: 75 to SEQ ID NO.: 98 for detecting the presence or absence in a bovine subject of at least one genetic marker associated with resistance to mastitis according to the method of claim 1.


**Patentansprüche**

**1.** Verfahren zum Bestimmen der Resistenz gegen Mastitis in Versuchsrindern des Roten Dänischen Milchrinds, Ayrshire-Rinds, Schwedischen Rot-Weißviehs und Holstein-Rinds, umfassend Detektieren des Vorhandenseins oder Fehlens wenigstens eines genetischen Markers in einer Probe von dem Versuchsrind, der mit wenigstens einem Merkmal verbunden ist, das für eine Mastitisresistenz bezeichnend ist, wobei der wenigstens eine genetische Marker auf dem bovinen Chromosom BTA9 in dem Bereich lokalisiert ist, der die polymorphen Mikrosatellitenmarker C6orf93 und inra084 beinhaltet und durch diese flankiert ist, wobei das Vorhandensein oder Fehlen des wenigstens einen genetischen Markers für eine Mastitisresistenz des Versuchsrinds oder eines Nachkommens desselben bezeichnend ist.

**2.** Verfahren nach Anspruch 1, wobei der wenigstens eine genetische Marker in dem Bereich lokalisiert ist, der die genetischen Marker bms2251 und inra084 des BTA9 beinhaltet und durch diese flankiert ist.

**3.** Verfahren nach Anspruch 1, wobei der wenigstens eine genetische Marker in dem Bereich lokalisiert ist, der die genetischen Marker bm7234 und inra144 des BTA9 beinhaltet und durch diese flankiert ist.

**4.** Verfahren nach Anspruch 1, wobei der wenigstens eine genetische Marker in dem Bereich lokalisiert ist, der die genetischen Marker bms2819 und inra144 des BTA9 beinhaltet und durch diese flankiert ist.

**5.** Verfahren nach Anspruch 1, wobei der wenigstens eine genetische Marker in dem Bereich lokalisiert ist, der die genetischen Marker bm4208und inra144 beinhaltet und durch diese flankiert ist.

**6.** Verfahren nach Anspruch 1, wobei der wenigstens eine genetische Marker der Mikrosatellitenmarker C6orf93 ist.

**7.** Verfahren nach Anspruch 1, wobei der wenigstens eine genetische Marker der Mikrosatellitenmarker DIK4986 ist.

**8.** Verfahren nach Anspruch 1, wobei der wenigstens eine genetische Marker der Mikrosatellitenmarker mm12e6 ist.

**9.** Verfahren nach Anspruch 1, wobei der wenigstens eine genetische Marker der Mikrosatellitenmarker PEX3 ist.

**10.** Verfahren nach Anspruch 1, wobei der wenigstens eine genetische Marker der Mikrosatellitenmarker DEAD21 ist.

**11.** Verfahren nach Anspruch 1, wobei der wenigstens eine genetische Marker der Mikrosatellitenmarker BMS 2251 ist.

**12.** Verfahren nach Anspruch 1, wobei der wenigstens eine genetische Marker der Mikrosatellitenmarker EPM2A ist.

**13.** Verfahren nach Anspruch 1, wobei der wenigstens eine genetische Marker der Mikrosatellitenmarker BM7234 ist.

**14.** Verfahren nach Anspruch 1, wobei der wenigstens eine genetische Marker der Mikrosatellitenmarker BM4208 ist.

**15.** Verfahren nach Anspruch 1, wobei der wenigstens eine genetische Marker der Mikrosatellitenmarker BMS2819 ist.

**16.** Verfahren nach Anspruch 1, wobei der wenigstens eine genetische Marker der Mikrosatellitenmarker INRA144 ist.

**17.** Verfahren nach Anspruch 1, wobei der wenigstens eine genetische Marker der Mikrosatellitenmarker INRA084 ist.

**18.** Verfahren nach Anspruch 1, wobei der wenigstens eine genetische Marker eine Kombination von genetischen Markern ist.

**19.** Verfahren nach Anspruch 6, wobei das Primerpaar zum Amplifizieren des Mikrosatellitenmarkers C6orf93 SEQ ID NO.: 75 und SEQ ID NO.: 76 ist.

**20.** Verfahren nach Anspruch 9, wobei das Primerpaar zum Amplifizieren des Mikrosatellitenmarkers PEX3 SEQ ID NO.: 81 und SEQ ID NO.: 82 ist.

**21.** Verfahren nach Anspruch 12, wobei das Primerpaar zum Amplifizieren des Mikrosatellitenmarkers EPM2A SEQ ID NO.: 87 und SEQ ID NO.: 88 ist.

**22.** Verfahren nach Anspruch 1, wobei der wenigstens eine genetische Marker eine Kombination von genetischen Markern ist.

**23.** Verfahren zum Auswählen von Versuchsrindern für Zuchtzwecke, wobei das Verfahren das Verfahren nach Anspruch 1 zum Bestimmen der Resistenz gegen Mastitis umfaßt.

**24.** Verwendung eines diagnostischen Kits, umfassend wenigstens eine Oligonucleotidsequenz, wobei die Nucleotidsequenz aus einer von SEQ ID NO.: 75 bis SEQ ID NO.: 98 ausgewählt ist, um das Vorhandensein oder Fehlen wenigstens eines genetischen Markers, der in Zusammenhang mit einer Resistenz gegen Mastitis steht, nach dem Verfahren aus Anspruch 1 in einem Versuchsrind zu detektieren.

**Revendications**

**1.** Procédé permettant de déterminer une résistance à la mammite chez n'importe quel sujet bovin des races rouge danoise, ayrshire, pie rouge de Suède et holstein, comprenant la détection dans un échantillon provenant dudit sujet bovin de la présence ou de l'absence d'au moins un marqueur génétique qui est lié à au moins un trait indiquant une résistance à la mammite, où ledit au moins un marqueur génétique est localisé sur le chromosome bovin BTA9 dans la région flanquée par et comprenant les marqueurs microsatellites polymorphes C6orf93 et inra084, où la présence ou l'absence dudit au moins un marqueur génétique indique une résistance à la mammite dudit sujet bovin ou de sa descendance.

**2.** Procédé selon la revendication 1, dans lequel l'au moins un marqueur génétique est localisé dans la région flanquée par et comprenant les marqueurs génétiques bms2251 et inra084 du BTA9.

**3.** Procédé selon la revendication 1, dans lequel l'au moins un marqueur génétique est localisé dans la région flanquée par et comprenant les marqueurs génétiques bm7234 et inra144 du BTA9.

**4.** Procédé selon la revendication 1, dans lequel l'au moins un marqueur génétique est localisé dans la région flanquée par et comprenant les marqueurs génétiques bms2819 et inra144 du BTA9.

**5.** Procédé selon la revendication 1, dans lequel l'au moins un marqueur génétique est localisé dans la région flanquée par et comprenant les marqueurs génétiques bm4208 et inra144.

**6.** Procédé selon la revendication 1, dans lequel l'au moins un marqueur génétique est le marqueur microsatellite C6orf93.

**7.** Procédé selon la revendication 1, dans lequel l'au moins un marqueur génétique est le marqueur microsatellite DIK4986.

**8.** Procédé selon la revendication 1, dans lequel l'au moins un marqueur génétique est le marqueur microsatellite mm12e6.

9. Procédé selon la revendication 1, dans lequel l'au moins un marqueur génétique est le marqueur microsatellite PEX3.

10. Procédé selon la revendication 1, dans lequel l'au moins un marqueur génétique est le marqueur microsatellite DEAD21.

11. Procédé selon la revendication 1, dans lequel l'au moins un marqueur génétique est le marqueur microsatellite BMS 2251.

12. Procédé selon la revendication 1, dans lequel l'au moins un marqueur génétique est le marqueur microsatellite EPM2A.

13. Procédé selon la revendication 1, dans lequel l'au moins un marqueur génétique est le marqueur microsatellite BM7234.

14. Procédé selon la revendication 1, dans lequel l'au moins un marqueur génétique est le marqueur microsatellite BM4208.

15. Procédé selon la revendication 1, dans lequel l'au moins un marqueur génétique est le marqueur microsatellite BMS2819.

16. Procédé selon la revendication 1, dans lequel l'au moins un marqueur génétique est le marqueur microsatellite INRA144.

17. Procédé selon la revendication 1, dans lequel l'au moins un marqueur génétique est le marqueur microsatellite INRA084.

18. Procédé selon la revendication 1, dans lequel l'au moins un marqueur génétique est une combinaison de marqueurs génétiques.

19. Procédé selon la revendication 6, dans lequel la paire d'amorces pour l'amplification du marqueur microsatellite C6orf93 est SEQ ID NO : 75 et SEQ ID NO : 76.

20. Procédé selon la revendication 9, dans lequel la paire d'amorces pour l'amplification du marqueur microsatellite PEX3 est SEQ ID NO : 81 et SEQ ID NO : 82.

21. Procédé selon la revendication 12, dans lequel la paire d'amorces pour l'amplification du marqueur microsatellite EPM2A est SEQ ID NO : 87 et SEQ ID NO : 88.

22. Procédé selon la revendication 1, dans lequel l'au moins un marqueur génétique est une combinaison de marqueurs génétiques.

23. Procédé de sélection de sujets bovins pour des objectifs d'élevage, ledit procédé étant composé par le procédé selon la revendication 1 permettant de déterminer une résistance à la mammite.

24. Utilisation d'un kit de diagnostic comprenant au moins une séquence oligonucléotidique, où les séquences nucléotidiques sont choisies parmi n'importe lesquelles de SEQ ID NO : 75 à SEQ ID NO : 98 pour la détection de la présence ou de l'absence chez un sujet bovin d'au moins un marqueur génétique associé à une résistance à la mammite selon le procédé de la revendication 1.

Fig. 1

29

Data sccdk, Markers gdmrk59dk_delzerocol, Map map_59_dk, Show Test-F

Fig. 2

Data mastfin, Markers gdmrk59linux_fin_correct, Map map_59_fin, Show Test-F

Fig. 3

Data sccfin, Markers gdmrk59linux_fin_correct, Map map_59_fin, Show Test-F

Fig. 4

Fig. 5a

Fig. 5b

Fig. 6a

Fig. 6b

Data masthf, Markers gdmrk59hf_delzerocol, Map map_59_hf, Show Test-F

Fig. 7

EP 2 069 531 B1

Data scchf, Markers gdmrk59hf_delzerocol, Map map_59_hf, Show Test-F

Fig. 8

**Fig. 9**

**Fig. 10**

QTL Profile for MR in FA

Fig. 11

**QTL Profile for MR in combined DR&FIN**

Fig. 12

Fig. 13

QTL Profile for SCC in SR

Fig. 14

**Fig. 15**

Fig. 16

Fig. 17

**Fig. 18**

EP 2 069 531 B1

**Fig. 19**

Fig. 20

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **AUSUBEL F.M. et al.** Current Protocols in Molecular Biology. Green Publishing Associates, Inc., and John Wiley & sons, Inc, 1989, vol. I, 5.3.1-6.3.62.10.3